(19) Europäisches Patentamt
European Patent Office
Office européen des brevets

(11) Numéro de publication: **0 527 185 B1**

(12) # FASCICULE DE BREVET EUROPEEN

(45) Date de publication de fascicule du brevet: **12.04.95**

(51) Int. Cl.[6]: **C07F 9/6561**, A61K 31/675, C07F 9/40

(21) Numéro de dépôt: **91908988.8**

(22) Date de dépôt: **23.04.91**

(86) Numéro de dépôt internationale : **PCT/FR91/00335**

(87) Numéro de publication internationale : **WO 91/16330 (31.10.91 91/25)**

(54) **PRODROGUES D'AMETHOPTERINE(METHOTREXATE), PRODUITS INTERMEDIAIRES, PROCEDE DE PREPARATION ET COMPOSITIONS LES CONTENANT.**

(30) Priorité: **23.04.90 FR 9005303**

(43) Date de publication de la demande: **17.02.93 Bulletin 93/07**

(45) Mention de la délivrance du brevet: **12.04.95 Bulletin 95/15**

(84) Etats contractants désignés: **AT BE CH DE DK ES FR GB GR IT LI LU NL SE**

(56) Documents cités:
**WO-A-88/06158**

**COMPTES RENDUS DE L'ACADEMIE DES SCIENCES, tome 310, série II, no. 6, 15 mars 1990, Academie des Sciences, Paris (FR); G. STURTZ et al., pp. 739-742&NUM;**

(73) Titulaire: **BRITISH TECHNOLOGY GROUP LIMITED**
**101 Newington Causeway**
**London SE1 6BU (GB)**

(72) Inventeur: **STURTZ, Georges**
**22, rue Léon-Blum**
**F-29200 Brest (FR)**

(74) Mandataire: **Corlau, Vincent**
**c/o Cabinet Vidon,**
**Immeuble Germanium,**
**80 avenue des Buttes de Coesmes**
**F-35700 Rennes (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 527 185 B1

## Description

La présente invention a pour objet la synthèse d'analogues gem-diphosphoniques d'améthoptérine (méthotrexate), et leur utilisation à titre de compositions pharmaceutiques, pour différentes applications thérapeutiques.

L'invention concerne également les voies de préparation de tels composés.

Le méthotrexate est un agent antinéoplasique important. On a montré qu'il possède à la fois des propriétés immuno-dépressives et anti-inflammatoires bien que son mécanisme d'action dans ces affections ne soit pas connu de façon précise. A faibles doses, il est couramment employé dans le traitement de rhumatismes psoriasiques rebelles, des polymyosites, de la maladie de Reiter et plus récemment des polyarthrites rhumatoïdes.

A fortes doses, il est également indiqué dans le traitement de certains cancers, notamment les leucémies de l'enfant, tumeurs trophoblastiques, cancers du sein, de la tête et du cou.

Cependant, l'intérêt comme agent anticancéreux est sous la dépendance limitante des importants effets secondaires que son action entraîne sur l'ensemble de l'organisme. Ceci a conduit plusieurs équipes de chercheurs à élaborer de nombreux analogues du méthotrexate dans le but de définir de meilleures relations structure-activité.

Dans cet esprit, le déposant a été amené, il y a quelques années, à développer un concept original de vectorisation pharmacologique d'agents antinéoplasiques vis à vis de carcinomes osseux, par des acides diphosphoniques.

Le tropisme osseux des acides diphosphoniques est en effet une caractéristique physiologique connue dans certaines applications thérapeutiques ou de diagnostic médical. (*SIRIS,E.S. ; CANFIELS,R.E. ; JACOBS,T.P. ; STODDART,KE. ; SPECTOR,P.J.* Met. Bone Dis. rel. Res. 1981, 4-5, 301).

Outre leur action remarquable dans la maladie de Paget (*EISENHUT,M.,Barder,J., TAYLOR,D.M.* Appl. radiat. Isot. 1978, 7, 535), ou dans l'arthrite rhumatoïde (*BRELIERE,J.C,EDMONDS-ALT,X., GARCIA,G.* - (Sanofi) Eur. Pat. Appl.), certains d'entre eux sont également utilisés en scintigraphie osseuse (*GASTRONOVO,F.P.; CALLAHAN, R.J.* J.Nucl.Med. 1972, 13,823).

Dans le cadre de l'étude de validité de ce concept, le déposant a décrit dans la demande de brevet française n°2611203 du 20.02.87 (inventeurs cités : STURTZ et VOISIN) et dans les comptes-rendus de l'Académie des Sciences, tome 310, série II, n°6, 15.03.90, p.739-742, la préparation et l'activité biologique des analogues diphosphoniques d'améthoptérine 3a et 3b :

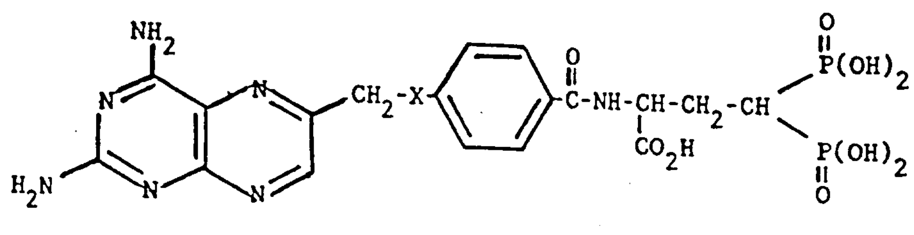

avec :
3a : X =

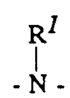

avec $R^1$ = H, alkyles inférieurs ($C_1$ à $C_6$)
3b : X =

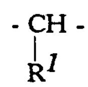

avec avec $R^7$ = H, alkyles inférieurs ($C_1$ à $C_6$)

Les résultats préliminaires des tests biologiques obtenus pour ces composés sur des modèles de cancer des os laissant apparaître un intérêt certain pour ces composés, le déposant a eu l'idée, à l'origine de la présente invention, d'associer ce concept de vectorisation à celui de prodrogue.

D'une manière générale, l'aspect prodrogue est recherché, de façon à favoriser l'atteinte sélective de la cellule cible, en évitant d'éventuels effets toxiques de la substance active sur d'autres sites du corps. A cette fin, et selon l'invention, il a été synthétisé des composés gem-diphosphoniques d'améthoptérine (méthotrexate) et de déaza-N-10 améthoptérine se présentant sous la forme d'une prodrogue d'améthoptérine dont le groupement gem-diphosphonique est relié au groupement ptéroylglutamique par une liaison peptidique.

Ces composés nouveaux présentent ainsi notamment l'avantage que la liaison peptidique peut être facilement rompue par hydrolyse. En conséquence, lorsqu'ils sont utilisés à titre de compositions pharmaceutiques, ces composés combinent l'effet de vectorisation des composés diphosphoniques avec l'avantage d'obtenir directement l'acide ptéroylglutamique par simple hydrolyse de la prodrogue. Ceci favorise ensuite notamment la formation de dérivés polyglutamiques du méthotrexate, qui interviennent dans les effets cytotoxiques vis à vis des cellules tumorales ciblées.

Les composés diphosphoniques de l'invention répondent à la formule générale

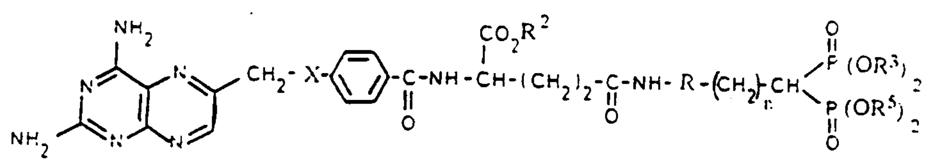

avec : $n \geq 0$, préférentiellement n = 0, 1, 2, 3

R étant soit absent, soit constitué par un groupement

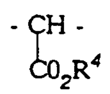

X =

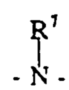 ou 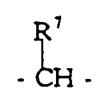

$R^7$, $R^3$, $R^4$, $R^5$ = H, ou alkyles inférieurs ($C_1$ à $C_6$)

$R^2$ = H, ou alkyles inférieurs ($C_1$ à $C_6$) et benzyle quand R présent

ainsi que les sels et les esters dérivés desdits composés.

Les composés diphosphoniques 1,2 suivants ont notamment été préparés :

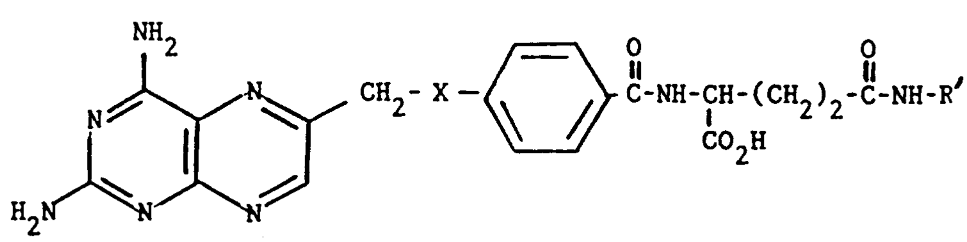

| | X | R' |
|---|---|---|
| <u>1a</u> | $-N(R^1)-$ | $-CH-(CO_2H)-CH_2-CH-[P(O)(OH)_2]_2$ |
| <u>1b</u> | $-CH(R^1)-$ | |
| <u>2a</u> | $-N(R^1)-$ | $-CH_2-CH-[P(O)(OH)_2]_2$ |
| <u>2b</u> | $-CH(R^1)-$ | |

. avec $R^1$ = H, alkyles inférieurs ($C_1$ à $C_6$).

L'utilisation de ces composés pourra également aisément être effectuée tant au niveau des esters ou des sels des acides correspondants.

On notera qu'il est attendu notamment une bonne efficacité du composé <u>1</u>, du fait de la présence du groupement carboxyméthyle, fréquent dans les enchaînements peptidiques, et susceptible de jouer un rôle favorable dans la fragilisation de la liaison peptidique du composé.

Sans exclure que ces composés, et l'ensemble de ceux de la famille générale définie plus haut présentent d'autres applications avantageuses, l'invention vise notamment à leur application en tant que constituants de compositions pharmaceutiques.

De telles compositions sont notamment utilisables en cancérologie, notamment pour le traitement de tumeurs ou métastases notamment osseuses, et/ou en rhumatologie, notamment pour le traitement de rhumatismes psoriasiques rebelles, de polymyosites, de la maladie de Reiter et de polyarthrites rhumatoïdes.

L'invention porte également sur des modes de préparation préférentiels des structures 1a,b et 2a,b, ainsi que sur les produits intermédiaires intéressants obtenus. Certains de ces produits intermédiaires, référencés ci-après 4a, 4b, 5a, 5b, 6a, 7a, 9 et 10 sont en outre susceptibles de présenter des propriétés intrinsèques intéressantes, hormis leur rôle dans la synthèse des prodrogues de l'invention.

Les molécules de types <u>a</u> et <u>b</u> ont fait l'objet d'une approche synthétique différente dans leur phase finale de préparation et sont donc présentées séparément ci-après. Ils sont obtenus, in fine, sous forme de sels de sodium.

En ce qui concerne les composés <u>1a</u>, <u>2a</u> leur obtention résulte de l'action sur les formes <u>4a</u> et <u>5a</u> :

- premièrement soit du bromure de triméthylsilyle suivie d'une méthanolyse, soit encore par exemple d'une action de coupure par l'acide bromhydrique dans l'acide acétique ;

- deuxièmement de la soude.

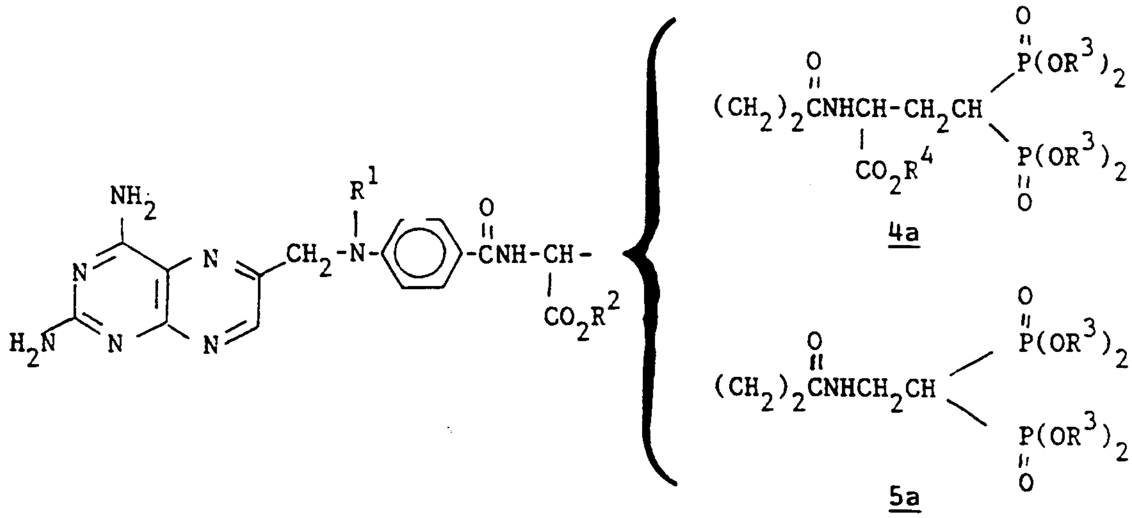

4a

5a

$R^1$ = H, alkyles inférieurs ($C_1$ à $C_6$) $R^3$ = alkyles inférieurs ($C_1$ à $C_6$) $R^2$ = alkyles inférieurs ($C_1$ à $C_6$) $R^2$ = benzyle pour 4a $R^4$ = alkyles inférieurs ($C_1$ à $C_6$)

Le dérivé bromé intermédiaire 8 est isolé en traitant la 2,4-diamino-6-hydroxyméthylptéridine préparée selon un protocole décrit par ROSOWSKY dans le Journal of Medicinal Chemistry, 28, 660-667 (1985) par le bromotriphénylphosphorane selon PIPER et MONTGOMERY (Journal of Heterocyclic Chemistry, 11 , 279-280, (1974).

Ces esters diphosphoniques 4a et 5a ont purifiés par recristallisation dans un mélange chloroforme/éther avec un rendement de l'ordre de 70 %.

L'étape précédente qui, suivant l'invention, permet d'obtenir les composés 6a et 7a consiste en la condensation d'un acide p-N(alkylamino) benzoïque sur les dérivés diphosphoniques 9 et 10, synthons communs aux dérivés 1a,b et 2a,b.

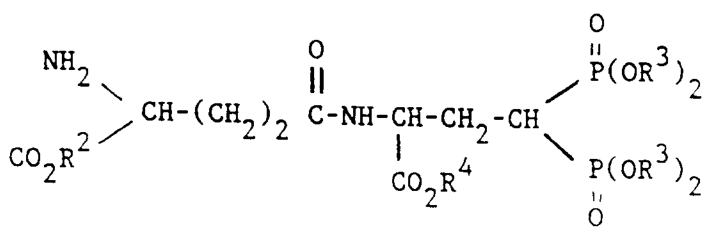

9

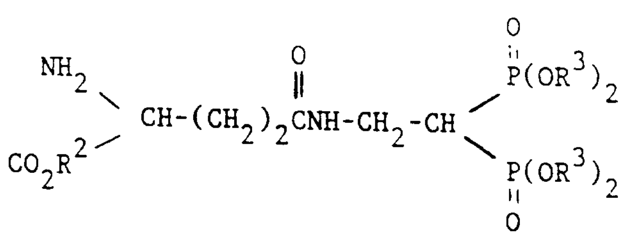

10

$R^2$ = alkyles inférieurs ($C_1$ à $C_6$) pour 10
$R^2$ = benzyle pour 9
$R^3$ = alkyles inférieurs ($C_1$ à $C_6$)
$R^4$ = alkyles inférieurs ($C_1$ à $C_6$).

Le procédé de l'invention pour la préparation de composés de types 6a et 7a propose, dans une première étape, de protéger l'acide p-N(alkyl amino) benzoïque par le 2,2,2-trichloroéthoxycarbonyle puis

d'activer sa fonction acide par le chlorure de thionyle pour former intermédiairement les composés :

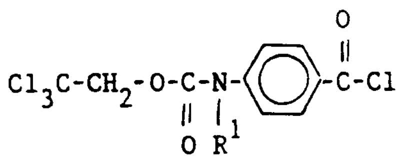

18

$R^1$ = H, alkyles inférieurs ($C_1$ à $C_6$)

La deuxième étape permet, par condensation de dérivés de types 9 et 10 dans le tétrahydrofurane en présence de triéthylamine, sur le chlorure d'acide benzoïque N-protégé, suivie d'une chromatographie sur colonne de gel de silice d'obtenir après libération de la fonction amine par la poudre de zinc, les dérivés de types 6a et 7a.

Les dérivés 1b et 2b , comme les composés 1a et 2a, sont issus de leurs analogues esters diphosphoniques 4b et 5b , après une hydrolyse des esters phosphoniques par le bromure de triméthylsilyle suivie d'une méthanolyse et une hydrolyse des esters carboxyliques par action de la soude.

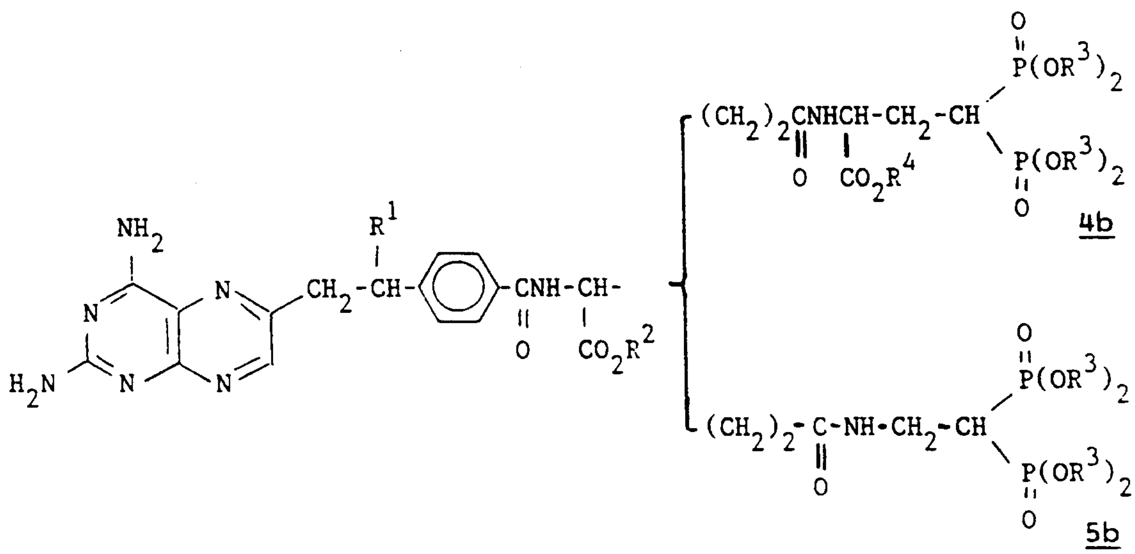

4b

5b

$R^1$ = H, alkyles inférieurs ($C_1$ à $C_6$)
$R^2$ = alkyles inférieurs ($C_1$ à $C_6$) pour 5b $R^2$ = benzyle pour 4b
$R^3$ = $R^4$ = alkyles inférieurs ($C_1$ à $C_6$).

En ce qui concerne les composés 4b et 5b ci-dessus, l'invention propose de les obtenir par une stratégie de synthèse totalement différente de celle utilisée pour les dérivés aza N-10 diphosphoniques de types a.

Le procédé de l'invention pour la préparation de composés déaza-10 aminoptérine repose en effet sur la condensation peptidique entre l'acide 4-amino-4-déoxy déaza N-10-ptéroïque 20 ou de ses dérivés alkylés en 10, dont les modes d'accès ont été décrits par exemple par PIPER et al, dans Journ. Med. Chem. 1980, 23, 320-321, et les synthons diphosphoniques de formules générales 9 et 10.

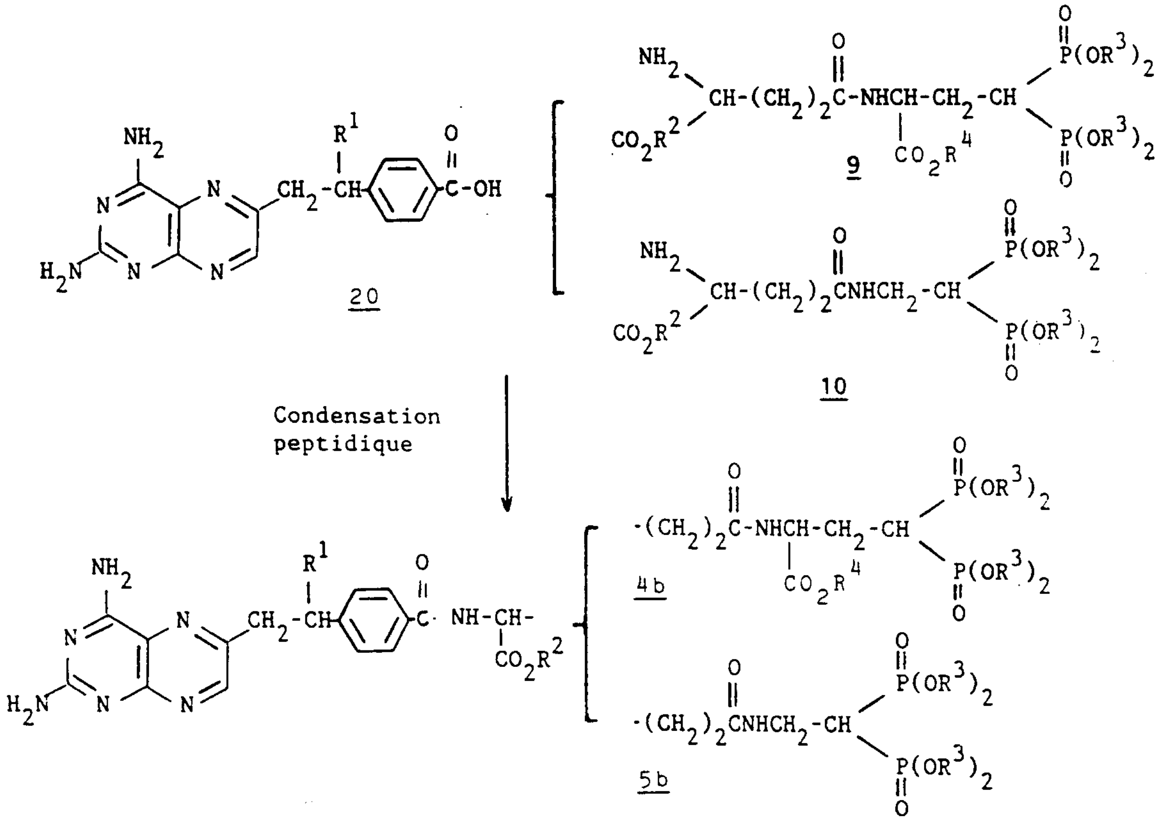


20 9 10 4b 5b

$R^1$ = H, alkyles inférieurs ($C_1$ à $C_6$)
$R^2$ = alkyles inférieurs ($C_1$ à $C_6$) pour 10 et 5b
$R^2$ = alkyles inférieurs ($C_1$ à $C_6$) pour 9 et 4b
$R^4$ = alkyles inférieurs ($C_1$ à $C_6$)

La littérature propose l'utilisation de différents agents de couplage pour réaliser ce type de condensation peptidique.

La méthode classique dite à "l'anhydride mixte" fait intervenir par exemple le chlorocarbonate d'isobutyle comme agent de couplage.

La réaction a lieu à 50 ° C, en présence de triéthylamine dans le N-N-diméthylformamide.

L'invention utilise cette méthode de couplage et l'applique, avec le chlorocarbonate d'isobutyle, à la synthèse des dérivés analogues diphosphoniques et de prodrogues déaza N-10 et déaza N-10 alkyles du Méthotrexate. Il est également possible d'utiliser toute méthode connue de l'homme du métier.

On notera que cette méthode de couplage est autant utilisable pour l'obtention des composés 4b, 5b, que pour l'obtention des composés 4a, 5a. Dans ce dernier cas, la condensation peptidique est effectuée entre les synthoms diphosphoniques 9 et 10 et l'acide 4 amino 4 déoxy aza-ptéroïque ou ses dérivés alkglés en 10.

Pour atteindre les composés de types 9, la présente invention propose de reprendre les travaux de SACHS et BRAND qui obtiennent dans le Journal of the American Chemical Society, 75, 4610-4611, (1953), l'ester α-benzylique de l'acide glutamique 12, après acidolyse sélective, en présence d'acide iodhydrique, du diester benzylique.

Avant de procéder au couplage peptidique de cet amino acide avec les dérivés 2-amino-4,4-bis-(dialkylphosphono)butyrate d'alkyles 11 en présence d'un des différents agents de couplage proposés dans la littérature, par exemple le dicyclohexylcarbodiimide, la fonction amine du composé 12 est protégée sous forme d'uréthane, par action du dicarbonate de tertiobutyle, par exemple.

STURTZ et VOISIN ont décrit dans le brevet français Fr 87 02449 fév. 1987
la synthèse des 2-amino-4,4-bis (dialkylphosphono) butyrate d'alkyles 11.

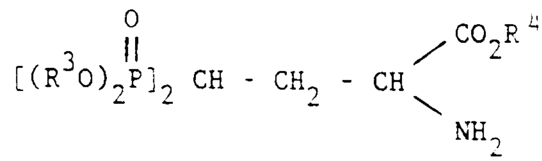

$R^4$ = $R^3$ = alkyles inférieurs ($C_1$ à $C_6$).

La coupure et la décarboxylation du groupement carbamique est réalisées par acidolyse dans l'acide trifluoroacétique. (TFA)

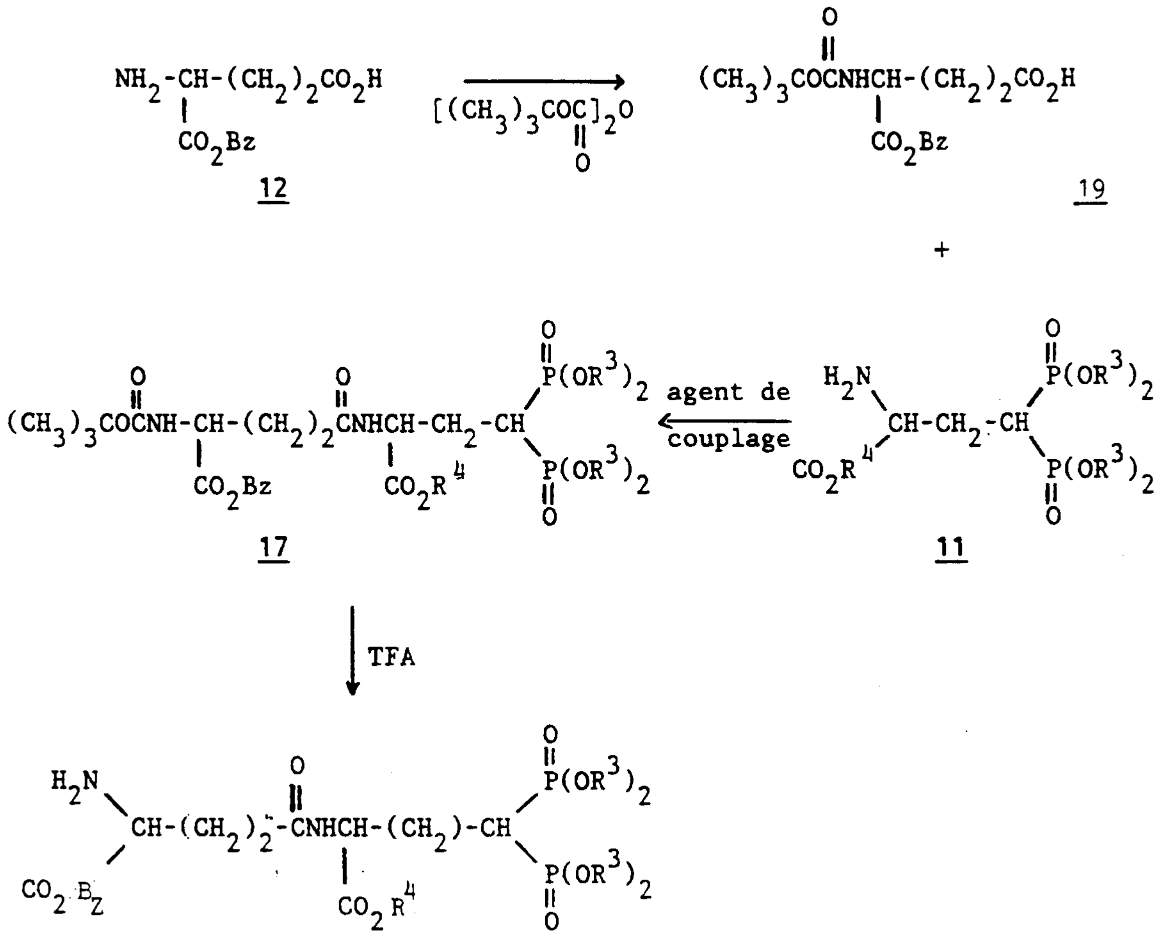


12 19 17 11 9

$R^2$ = $R^3$ = alkyles inférieurs ($C_1$ à $C_6$).

Les composés de type 10 sont obtenus selon l'invention par addition nucléophile, de type Michaël, de dérivés de la glutamine 13 sur les éthylidène bis(phosphonates) d'alkyles 14, suivie d'une déprotection de la fonction amine par l'acide trifluoroacétique.

Le composé 13 est avantageusement obtenu selon le mode opératoire décrit notamment par TARBELL et al, Proc. Nat. Acad. Sci. 1972, 69, 730.

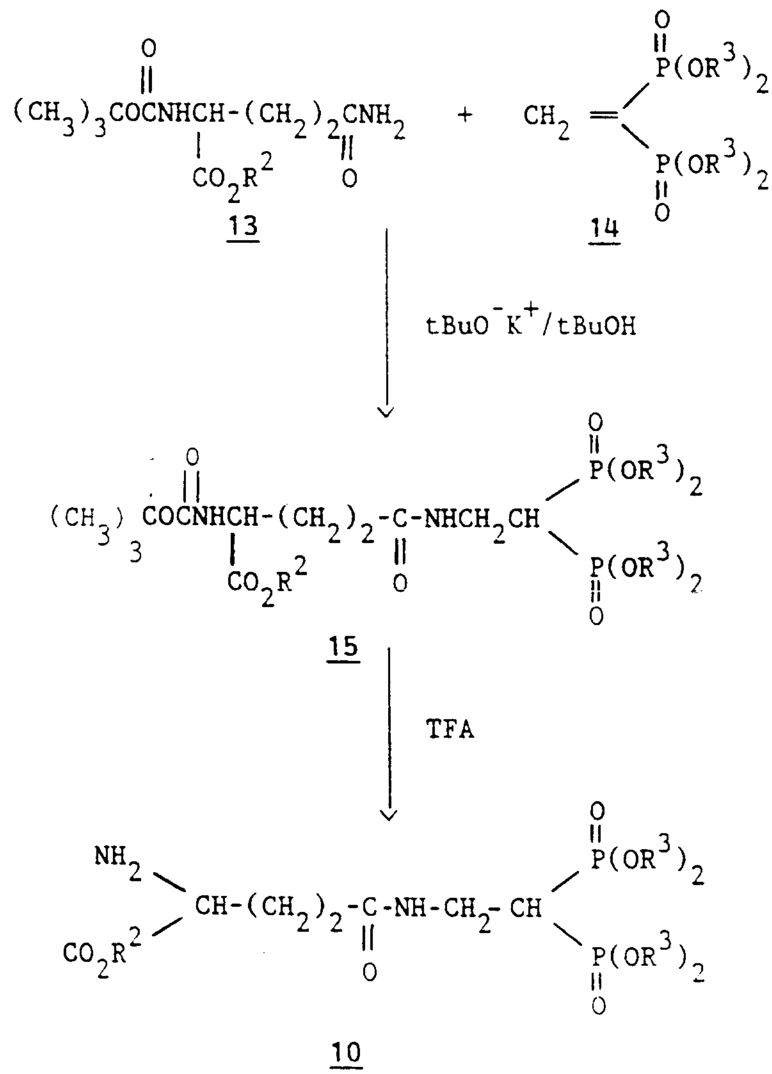


$R^2$ = $R^3$ = alkyles inférieurs ($C_1$ à $C_6$).

Dans l'invention, les éthylidènes bis(phosphonates) de dialkyles 14 sont issus d'une isomérisation d'ARBUSOV entre un trialkyl phosphite et un 1-bromovinylphosphonate de dialkyle, lui-même obtenu à partir d'un vinylphosphonate de dialkyle suivant le mode opératoire utilisé par BELBEOC'H, Thèse Doctorat Sciences Physiques, Université de Bretagne Occidentale, Brest.

EXEMPLE 1

Synthèse des composés 1a et 1b (où $R^1$ = $CH_3$)

a) N [(4(N-tertiobutyloxycarbonyl)amino-4-benzyloxycarbonyl) butanoyl]-2-amino-4,4 bis(diéthylphosphono)-butyrate d'éthyle.

Dans un tricol de 100 ml, muni d'un thermomètre à alcool, on place 2 g (5,9 mmoles) d'ester α-benzylique de l'acide N(tertiobutyloxycarbonyl)-L-glutamique dans 100 ml de chloroforme.

A cette solution refroidie à 0-5°C est additionné 2,5 g (6.2 mmoles) de 2-amino-4,4-bis-(diéthylphosphono)butyrate d'éthyle dilué dans 10 ml de $CHCl_3$.

Le mélange résultant est alors additionné goutte à goutte pendant 15 mn par une solution de 1.2 g (6.2 mmoles) de dicyclohexylcarbodiimide dans 20 ml de $CHCl_3$.

A la fin de l'addition, le bain de glace est enlevé et un important précipité de dicyclohexylurée apparait.

L'agitation à température ambiante est maintenue pendant 2 heures environ avant que le solvant ne soit évaporé sous pression réduite.

Le résidu est repris dans quelques ml d'acétate d'éthyle puis filtré sur cellite.

Le filtrat est récupéré, dilué puis lavé successivement par NaOH 5 %, HCl 1N et $H_2O$.

La phase organique finale, conduit, après séchage sur $Na_2SO_4$ et évaporation, à une huile visqueuse incolore (3,3 g ; 78 %).

RMN $^{31}P$ : $\delta P$ = 23. 2, s.

b) N-[(4-amino-4-benzyloxycarbonyl)butanoyl]-2-amino-4,4-bis (diéthylphosphono) butyrate d'éthyle.

Une solution de 17 (2.17 g ; 3 mmoles) dans 10 ml de $CH_2Cl_2$ sont additionnés goutte à goutte 15 ml d'acide trifluoroacétique, sans que ne soit décelée d'élévation de température.

La fin de la réaction est détectée après 20 mn, par la fin du dégagement de $CO_2$.

Les solvants sont éliminés par évaporation et le résidu repris dans 25 ml d'acétate d'éthyle.

La solution résultante est alors additionnée par une solution aqueuse de $NaHCO_3$ (jusque pH = 8-9), décantée puis extraite.

La phase organique est lavée à l'eau, séchée sur $Na_2SO_4$ et finalement évaporée pour donner le produit déprotégé attendu sous forme d'une huile incolore (1.7 g ; 90 %).

L'absence en RMN du $^1H$ du singulet à 1.5 ppm (/TMS) correspondant aux protons du groupement tertiobutyloxycarbonyle confirme la forme amine libre du composé désiré.

RMN $^{31}P$ : $\delta P$ = 22.9, d, ($J_{P-P}$ = 2.5 Hz).

c) N[(4(N-tertiobutyloxycarbonyl)-amino-4-éthyloxycarbonyl)butanoyl] -2-amino-1,1-bis (diéthylphosphono)-éthane 15

Dans un tricol de 100 ml, muni d'un réfrigérant ascendant et d'un thermomètre à mercure, on dissout à 50°C 0,1 g de tBuOK dans 50 ml de tBuOH. 2.74 g (0,01 mole) du composé 13 sont alors ajoutés en un seul pot.

L'agitation est maintenue à 50°C jusqu'à totale dissolution. On additionne ensuite 3.1 g (0,0103 mole) de 14 goutte à goutte et l'agitation est conservée pendant 1 heure à cette température.

L'évolution de la réaction est contrôlée par RMN du $^{31}P$.

Le pH est enfin ajusté à 7 par addition d'une solution saturée de chlorure d'ammonium puis extraite par du chlorure de méthylène (2 fois).

Les phases organiques sont rassemblées, séchées sur $Na_2SO_4$ et évaporées sous pression réduite pour donner 5.2 g (90 % de rendement) d'un solide blanc.

RMN $^{31}P$ : $\delta P$ = 18.9, s.

d) N[(4-amino-4-éthyloxycarbonyl)butanoyl]-2-amino-1,1-bis(diéthylphosphono) éthane 10.

A 2.0 g (3.5 mmoles) de 15 dissous dans 15 ml de $CH_2Cl_2$ sont additionnés goutte à goutte 15 ml de TFA.

L'agitation du mléange réactionnel est maintenue pendant 20 mn à température ambiante puis les solvants sont évaporés.

Le résidu visqueux est repris par une solution aqueuse de $NaHCO_3$ (jusqu'à pH = 8-9) puis cette solution est extraite 2 fois par l'acétate d'éthyle.

Les phases organiques sont séchées puis évaporées pour conduire à une huile visqueuse incolore (1.2 g ; 70 %).

RMN $^{31}P$ : $\delta P$ = 19.6, s.

e) Condensation du chlorure de l'acide [N,N[(2,2,2-trichloroéthyloxycarbonyl)méthyl]-4-amino] benzoïque avec les composés 9 et 10.

Une solution de 9 (9.85 g ; 0.016 mole) dans 100 ml de THF, contenant 25 ml de triéthylamine distillée, est agitée à 0-5°C pendant que 6.5 g (0.018 mole) du chlorure d'acide benzoïque N-protégé sont ajoutés goutte à goutte durant 15 mn.

A la fin de l'addition, l'agitation est maintenue à température ambiante pendant 1 heure eviron.

Après filtration du mélange réactionnel et évaporation du chloroforme, l'huile visqueuse résultante est extraite par un mélange $H_2O/CH_2Cl_2$.

Après séchage sur $Na_2SO_4$ et évaporation, le résidu est purifié sur colonne de gel de silice (solvant d'élution : AE/E : 20/1).

Les fractions intéressantes sont rassemblées et évaporées pour donner un produit sirupeux incolore (10.4 g ; 70 %).
RMN $^{31}P$ : δP = 23.1 ppm, s.

Nous avons repris le même protocole expérimental pour condenser le composé 10 avec le dérivé benzoïque N-protégé. Après chromatographie sur colonne de gel de silice, le produit désiré est obtenu avec un rendement de 68 %.
RMN $^{31}P$ : δP = 23.2 ppm, s.

f) Elimination du groupement protecteur de la fonction amine.

. Composé 6a

8.4 g (0.0091 mole) du composé résultant de la condensation entre le produit 9 et le dérivé chloré benzoïque sont dissous dans 80 ml de THF puis 1.3 g de $KH_2PO_4$ dissous dans 13 ml d'eau distillée sont additonnés, suivis de 8 g de poudre de zinc (préalablement activé par HCl 5 %).

L'agitation est maintenue à la température du laboratoire pendant une journée.

L'évolution de la réaction est contrôlée par CCM de gel de silice (solvant d'élution : AE).

Après filtration du zinc et évaporation du solvant, le résidu est extrait par un mélange eau/chloroforme.

La phase organique est décantée, séchée sur $Na_2SO_4$ et évaporée sous pression réduite pour donner quantitativement une huile incolore.
RMN $^{31}P$ : δP = 23.7 ppm, q, ($J_{P-P}$ = 2.4 Hz).

. Composé 7a

Le mode opératoire est identique à celui décrit ci-dessus.
RMN $^{31}P$ : δP = 23.1 ppm, s.

g) Préparation des composés 4a et 5a

3.4 g (0.01 mole) de 8 . HBr et 8.3 g (0.011 mole) de 6a sont agités dans 40 ml de DMA à 50°-55°C pendant 4 heures (la solubilisation est obtenue après 1 heure). Après évaporation du DMA, le résidu marron foncé est repris dans l'eau et extrait au chloroforme (2 fois).

Les phases organiques sont rassemblées et séchées sur $Na_2SO_4$.

Après évaporation du chloroforme, le résidu semi-solide est recristallisé dans un mélange $CHCl_3/Et_2O$ pour conduire au composé 4a sous forme d'une poudre jaune clair avec un rendement de 61 %.
RMN $^{31}P$ : δP = 23.2 ppm, s.

. Composé 5a

Il a été obtenu avec un rendement de 65 % par le même protocole expérimental que celui développé ci-dessus.
RMN $^{31}P$ : δP = 23.5 ppm, d.

Ces 2 poudres jaunes ont été étudiées par CCM de gel de silice ($CHCl_3/CH_3OH$ : 4/1).

Les analyses spectroscopiques en RMN $^{1}H$, $^{31}P$ et $^{13}C$ indiquent une pureté tout-à-fait satisfaisante pour ces composés et ne nécessitant pas d'étapes supplémentaires de purification.

h) Hydrolyse des composés 4a et 5a

Dans un ballon de 100 ml, on dissout 4,6 g (0.005 mole) de 4a dans 50 ml de $CH_2Cl_2$. Après ajout en une seule fois de 5.4 ml (0.04 mole) de $BrSi(CH_3)_3$, le mélange réactionnel est agité pendant deux jours à température ambiante, puis les solvants sont éliminés.

Le résidu solide orangé est repris dans 100 ml de méthanol puis agité durant 30 mn.

Après évaporation du solvant, le résidu est dissous dans quelques ml de méthanol puis précipité par ajout d'éther.

Après refroidissement, la poudre jaune est filtré, lavé à l'éther puis séché sur $P_2O_5$ à 50°C (rendement : 90 %).

. L'hydrolyse du composé 5a est réalisée dans les mêmes conditions opératoires.

i) saponification des composés 4a et 5a.

. Composé 1a

A une solution méthanolique du composé 4a portée à reflux est additionnée goutte à goutte une solution aqueuse de soude (4 éq.).

Un précipité jaune clair apparait dès que le pH atteint une valeur de 7.

L'agitation est ensuite conservée pendant 4 heures à cette température.

Après une nuit de réfrigération, le précipité est filtré, lavé par de petites quantités de méthanol, et d'éther pour être finalement séché sur $P_2O_5$ à 50 °C.

RMN $^{31}P$ : $\delta P$ = 22.2 ppm, s.

. Composé 2a

Même mode opératoire que pour le composé 1a.

RMN $^{31}P$ : $\delta P$ = 22.7 ppm, d, ($J_{P-P}$ = 12.2 Hz).

EXEMPLE 2

Synthèse des composés 1b et 2b (où $R^1$ = H).

a) Couplage peptidique entre les composés 9 et 10 et l'acide déazaptéroïque 16.

Dans un tricol de 100 ml, muni d'une agitation magnétique, on place 1.55 g (0.005 mole) d'acide déazaptéroïque 1b, 1.4 ml (0.01 mole) d'$Et_3N$ fraîchement distillée dans 50 ml de DMA anhydre.

On additionne dans un premier temps, 0.65 ml (0.005 mole) de chloroformate d'isobutyle pour former l'anhydride mixte. Après 15 mn d'agitation à température ambiante, on ajoute 3.1 g (0.005 mole) d'aminoester 9 et une minute plus tard à nouveau 0.33 ml de chloroformate d'isobutyle.

Au bout de 15 mn, 1.6 g (0.0025 mole) de 9 sont additionnés, suivis de 0.16 ml de chloroformate d'isobutyle. Après un délai de 15 mn, on ajoute à nouveau 0.8 g de 9 puis une minute plus tard 0.16 ml de réactif de couplage. 0.8 g d'ester 9 sont finalement ajoutés au bout d'un quart d'heure.

Un suivi de la réaction par chromatographie sur couches minces de gel de silice (solvant d'élution : $CHCl_3/CH_3OH$ : 4/1) montre l'apparition quasi-immédiate d'un pic différent de l'acide déazaptéroïque introduit.

La solution marron foncée est agitée à 50 °C pendant 4 heures environ avant que le DMA ne soit évaporé, sous pression réduite (P < 1 mmHg ; T° < 50 °C).

Le résidu marron sirupeux est repris dans l'eau (50 ml) et extrait par du chloroforme (2 x 100 ml). Les phases organiques sont rassemblées, séchées sur $Na_2SO_4$ puis évaporées.

Le semi-solide résultant est recristallisé dans un mélange chloroforme/éther.

Après quelques heures de refroidissement, les cristaux jaunes sont filtrés, lavés à l'éther puis séchés sur $P_2O_5$ à l'étuve sous vide à 60 °C pour donner le composé désiré 4b avec un rendement de 65 %.

RMN $^{31}P$ : $\delta P$ = 23.4, s.

. Composé 5b

Le couplage peptidique du composé 16 avec l'amino ester 10 est réalisé dans les mêmes conditions opératoires que celles développées ci-dessus.

Le composé 5b est obtenu avec un rendement de 60 % sous forme d'une fine poudre ocre claire.

b) Hydrolyse des fonctions esters carboxyliques et phosphoniques des composés 4b et 5b

Le bromotriméthylsilane est utilisé pour hydrolyser les esters phosphoniques (voir EXEMPLE 1 ; h) et la saponification est réalisée par une solution de soude dans le méthanol (voir EXEMPLE 1 ; i).

. Composé <u>1b</u>

RMN $^{31}P$ : $\delta P$ = 21.4, s.

. Composé <u>2b</u>

RMN $^{31}P$ : $\delta P$ = 19.8, <u>s</u>.

**Revendications**

**Revendications pour les Etats contractants suivants : AT, BE, CH, DE, DK, FR, GB, IT, LI, LU, NL, SE**

1. Composés gem-diphosphoniques d'améthoptérine (méthotrexate) et de déaza N-10 améthoptérine caractérisés en ce qu'ils se présentent sous la forme d'une prodrogue d'améthoptérine dont le groupement gem-diphosphonique est relié au groupement ptéroylglutamique par une liaison peptidique et répondant à la formule :

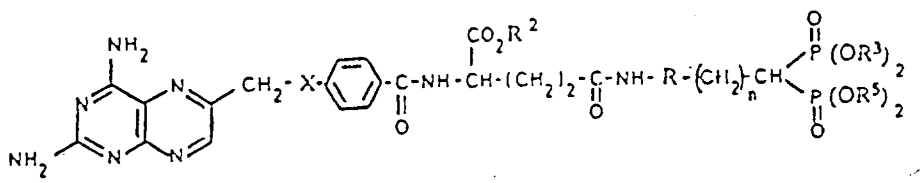

<u>avec</u> : n ≧ 0, préférentiellement n = 0, 1, 2, 3
R étant soit absent, soit constitué par un groupement

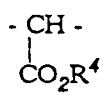

X =

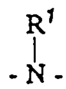 ou 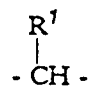

$R^1$, $R^3$, $R^4$, $R^5$ = H, ou alkyles inférieurs ($C_1$ à $C_6$)
$R^2$ = H, ou alkyles inférieurs ($C_1$ à $C_6$) et benzyle quand R présent ;
ainsi que les sels et les esters dérivés desdits composés.

2. Composés selon la revendication 1 dans lesquels X signifie

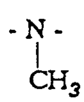

ou $-CH_2-$

3. Composition pharmaceutique, caractérisée en ce qu'elle contient comme ingrédient actif, au moins un composé selon l'une quelconque des revendications 1 ou 2.

4. Composition pharmaceutique selon la revendication 3 utilisable en cancérologie, notamment pour le traitement de tumeurs ou métastases notamment osseuses, et/ou en rhumatologie, notamment pour le

traitement de rhumatismes psoriasiques rebelles, de polymyosites, de la maladie de Reiter et de polyarthrites rhumatoïdes.

**5.** Composés gem-diphosphoniques utilisables notamment comme produits intermédiaires, de formule :

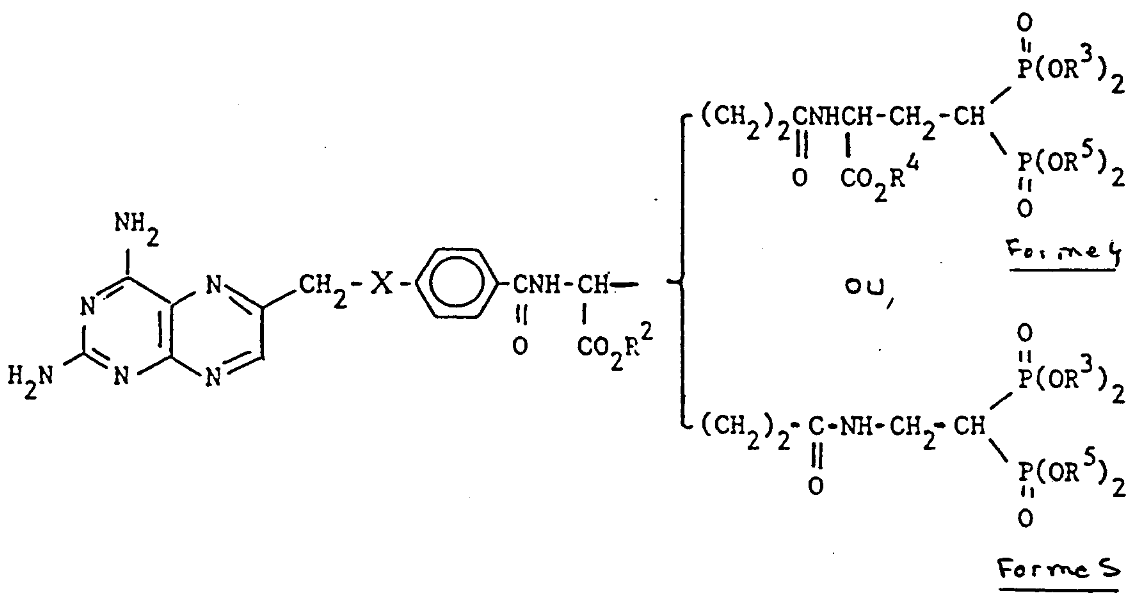

Forme 4

ou,

Forme 5

avec : X =

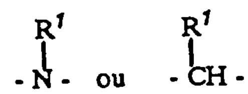

ou

$R^1$ = H, ou alkyles inférieurs ($C_1$ à $C_6$)
$R^2$ = alkyles inférieurs ($C_1$ à $C_6$) pour la forme 5; benzyle pour la forme 4 ;
$R^3$, $R^4$, $R^5$ : alkyles inférieurs ($C_1$ à $C_6$);
ainsi que leurs acides et sels dérivés.

**6.** Composés gem-diphosphoniques, utilisables notamment comme produits intermédiaires, de formule :

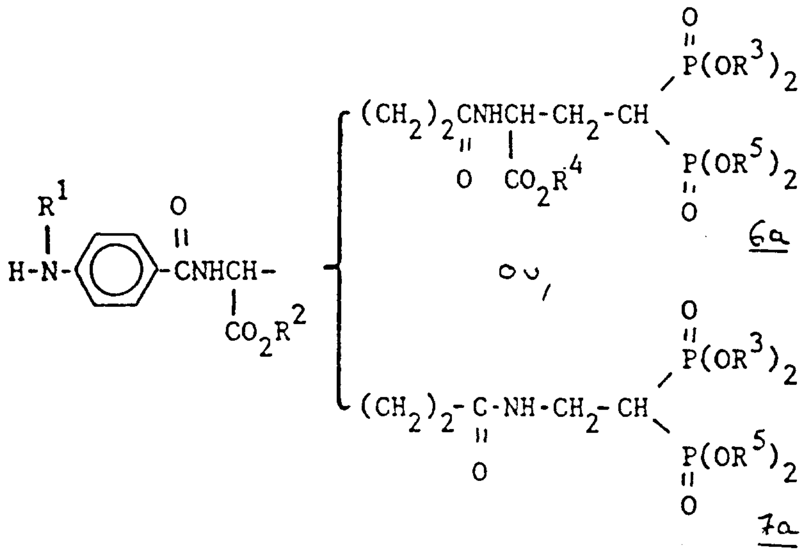

avec : $R^1$ = H, ou alkyles inférieurs ($C_1$ à $C_6$).
$R^3$, $R^4$, $R^5$ = alkyles inférieurs ($C_1$ à $C_6$)
$R^2$ = alkyles inférieurs ($C_1$ à $C_6$) pour 7a, benzyle pour 6a ;
ainsi que leurs acides et sels dérives.

**7.** Composés gem-diphosphoniques, utilisables notamment comme produits intermédiaires, de formule :

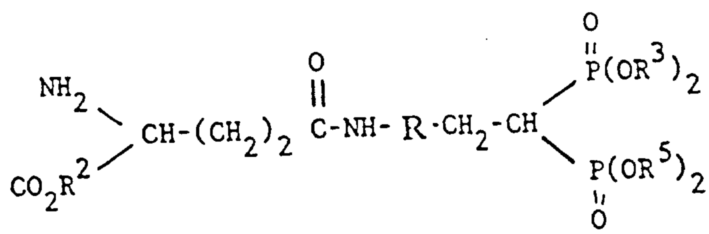

avec : R = absent ou

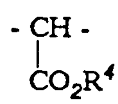

$R^2$ = H, ou alkyles inférieurs ($C_1$ à $C_6$) ; benzyle quand R présent
$R^3$, $R^4$, $R^5$ : alkyles inférieurs ($C_1$ à $C_6$) ;
ainsi que leurs acides et sels dérivés.

**8.** Procédé d'obtention d'un composé selon la revendication 7, de formule :

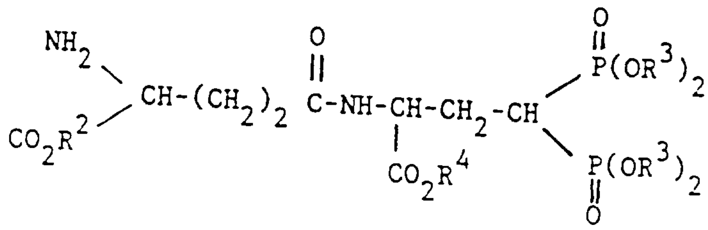

caractérisé en ce qu'il consiste à :

- protéger la fonction amine de l'ester α - benzylique de l'acide glutamique, sous forme d'uréthane
- procéder au couplage peptidique dudit amino-acide avec des dérivés 2-amino-4,4-bis-(dialkylphosphono)butyrate d'alkyles en présence d'un agent de couplage tel que le dicyclohexylcarbodiimide,
- déprotéger la fonction amine par acidolyse dans l'acide trifluoracétique.

**9.** Procédé d'obtention d'un composé selon la revendication 7 de formule:

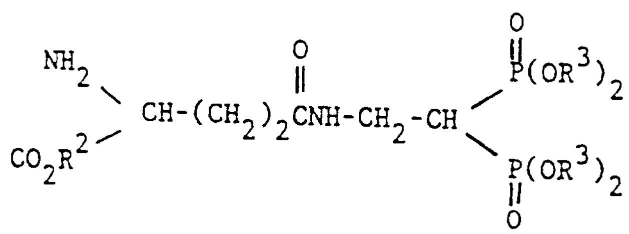

caractérisé en ce qu'il consiste à réaliser une addition nucléophile de type Michaël d'un amide primaire de formule:

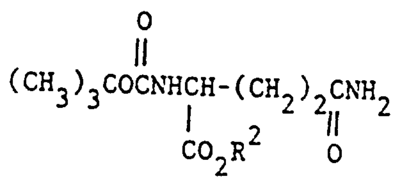

sur le vinylidène disphophonate de tétraalkyle de formule:

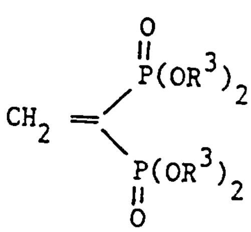

**10.** Procédé d'obtention d'un composé selon la revendication 5 de formule:

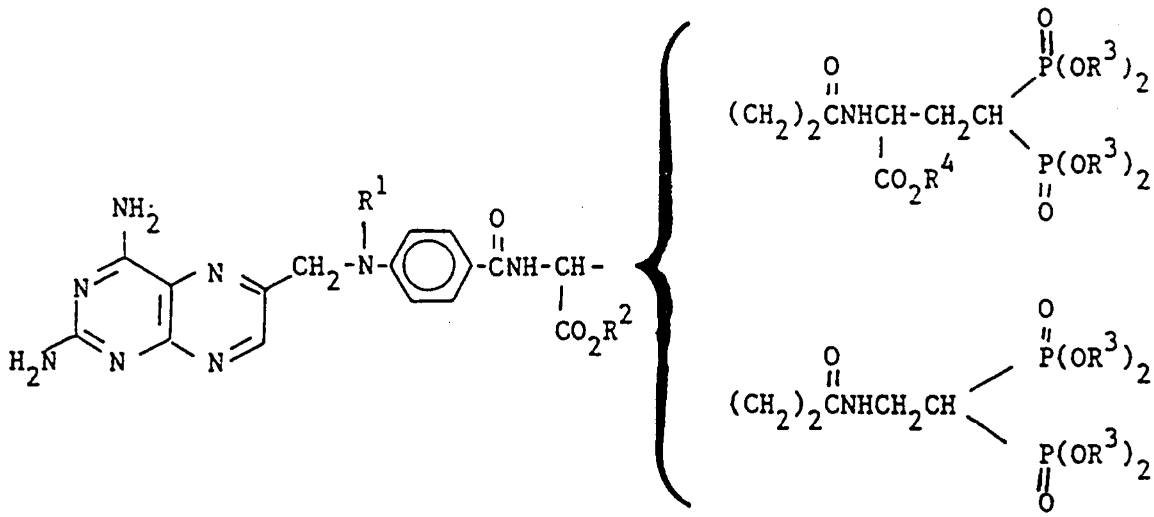

ou de formule

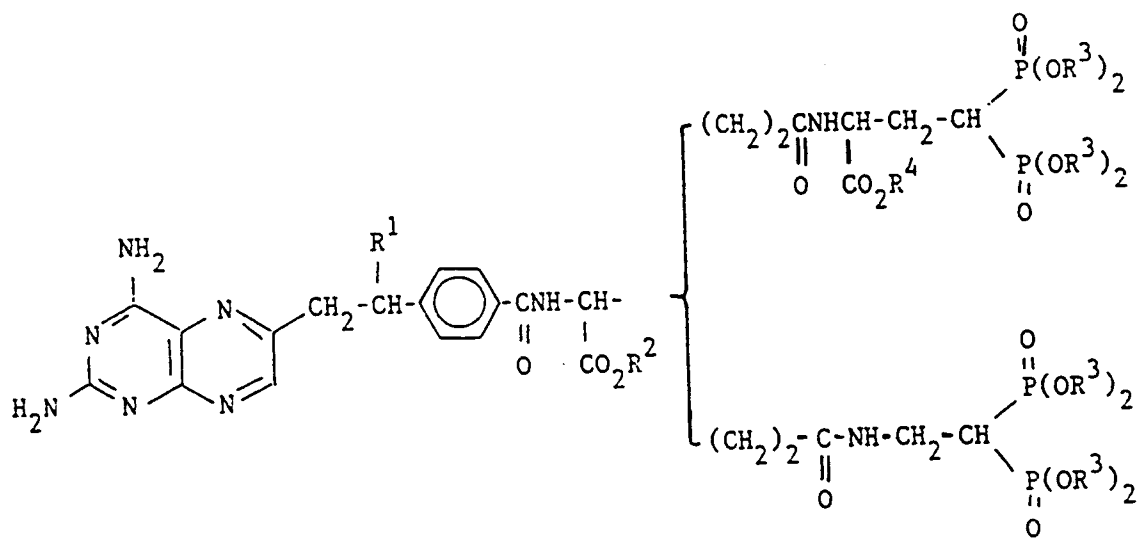

caractérisé en ce qu'il consiste à réaliser une condensation peptidique entre les synthons diphosphoniques de la revendication 7 et l'acide 4-amino-4-déoxy aza-ptéroïque ou ses dérivés alkylés en 10; ou l'acide 4-amino-4 déoxy déaza-N-10 ptéroïque ou ses dérivés alkylés en 10 respectivement.

**11.** Procédé d'obtention d'un composé selon la revendication 5 de formule,

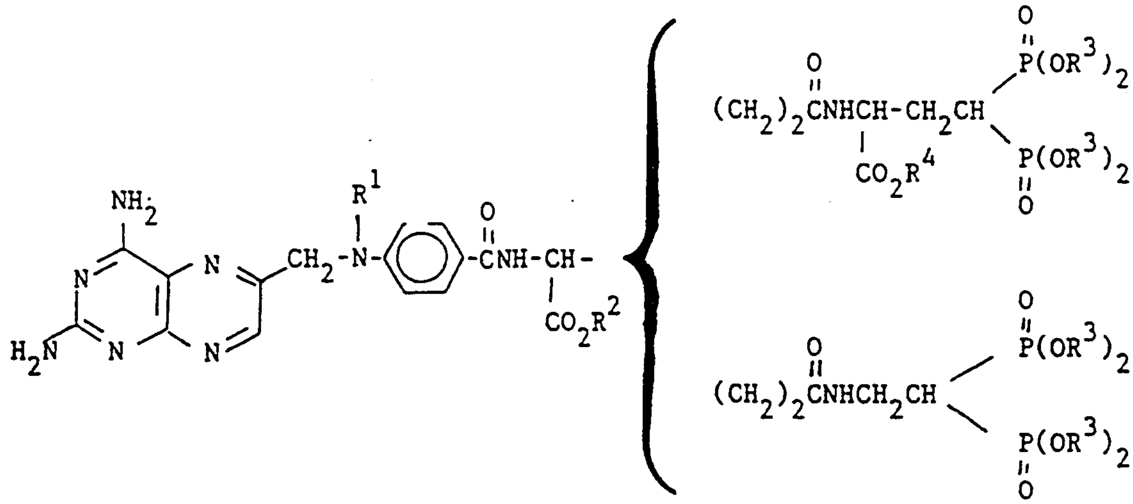

caractérisé en ce qu'il consiste à réaliser une substitution nucléophile en présence de solvant sur un composé selon la revendication 6 avec le 6-bromométhyl-2,4-diaminoptéridine.

**12.** Procédé d'obtention d'un composé selon la revendication 2 caractérisé en ce qu'il consiste à obtenir au moins un des produits intermédiaires des revendications 5 à 7 en utilisant au moins un des procédés des revendications 8 à 11.

**13.** Utilisation d'un composé gem-diphosphonique(méthotrexate) ou déaza-N-10-améthoptérine pour la fabrication d'un médicament utilisé comme agent immuno-dépresseur ou anti-inflammatoire.

**Revendications pour l'Etat contractant suivant : ES**

**1.** Procédé de préparation de composés gem-diphosphoniques d'améthoptérine (méthotrexate) et de déaza N-10 améthoptérine se présentant sous la forme d'une prodrogue d'améthoptérine dont le groupement gem-diphosphonique est relié au groupement ptéroylglutamique par une liaison peptidique et répondant à la formule :

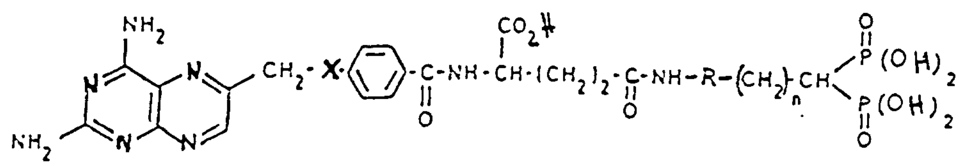

caractérisé en ce qu'il comprend les étapes consistant à faire agir sur un produit intermédiaire :

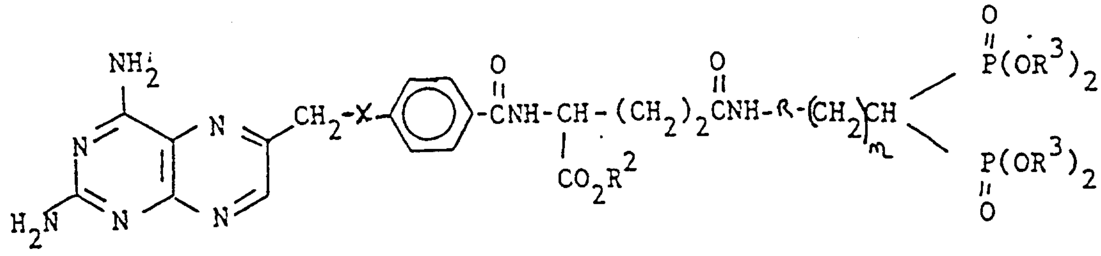

premièrement, du bromure de triméthylsilyle suivie d'une méthanolyse, pour hydrolyser les esters phosphoniques,
deuxièmement, de la soude, pour hydrolyser les esters carboxyliques, avec dans les formules
n ≧ 0, préférentiellement n = 0, 1, 2, 3
R étant soit absent, soit constitué par un groupement

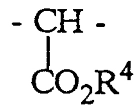

X =

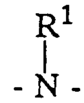

ou

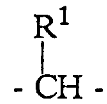

$R^1$, $R^3$, $R^4$, $R^5$ = H, ou alkyles inférieurs ($C_1$ à $C_6$)
$R^2$ = H, ou alkyles inférieurs ($C_1$ à $C_6$) et benzyle quand R présent.

**2.** Procédé de préparation de composés gem-diphosphoniques d'améthoptérine (méthotrexate) et de déaza N-10 améthoptérine se présentant sous la forme d'une prodrogue d'améthoptérine dont le groupement gem-diphosphonique est relié au groupement ptéroylglutamique par une liaison peptidique et répondant à la formule :

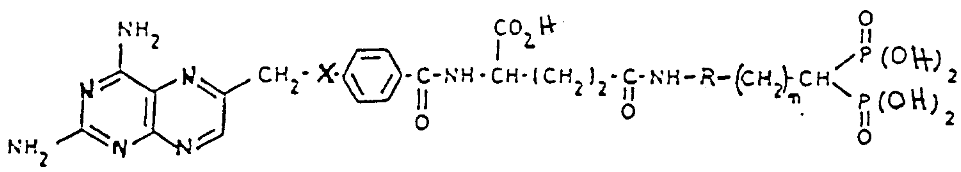

caractérisé en ce qu'il comprend les étapes consistant à faire agir sur un produit intermédiaire de formule :

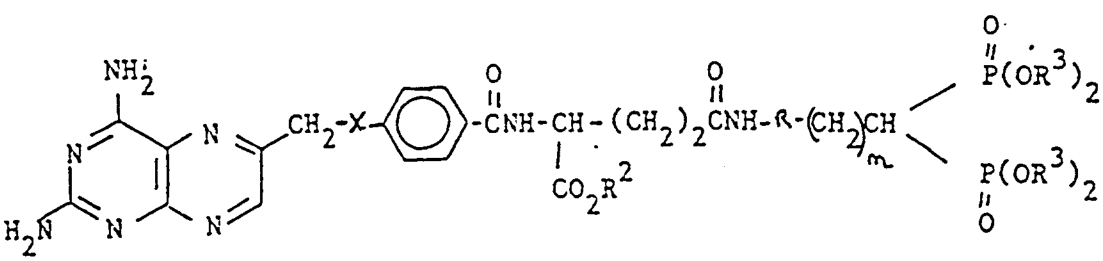

premièrement, de l'acide bromhydrique en présence d'acide acétique pour hydrolyser les esters phosphoniques ;
deuxièmement, de la soude, pour hydrolyser les esters carboxyliques,
avec dans les formules
n ≧ 0, préférentiellement n = 0, 1, 2, 3
R étant soit absent, soit constitué par un groupement

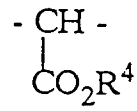

X =

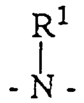 ou 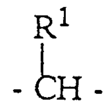

$R^1$, $R^3$, $R^4$, $R^5$ = H, ou alkyles inférieurs ($C_1$ à $C_6$)
$R^2$ = H, ou alkyles inférieurs ($C_1$ à $C_6$) et benzyle quand R présent.

3. Procédé selon la revendication 1 ou 2 dans lequel
X signifie

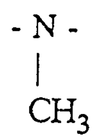

ou - $CH_2$ -

4. Procédé de préparation d'un intermédiaire de formule :

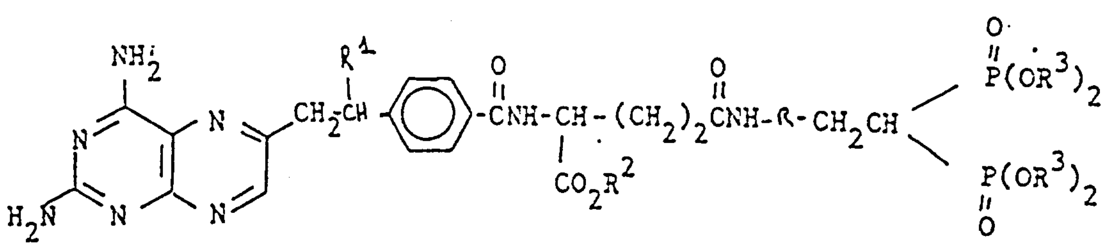

pour la mise en oeuvre du procédé selon l'une des revendications 1, 2 ou 3 caractérisé en ce qu'il consiste à réaliser une condensation peptidique entre un synthon diphosphonique de formule :

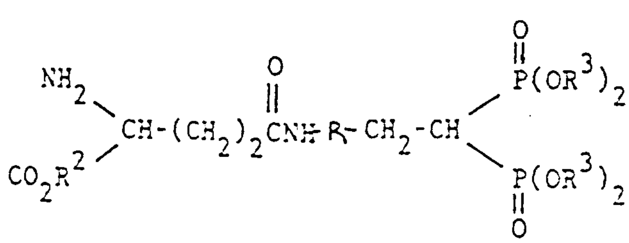

et l'acide 4-amino-4-déoxy-déaza-N-ptéroïque ou ses dérivés alkylés en 10.

**5.** Procédé de préparation d'un intermédiaire de formule :

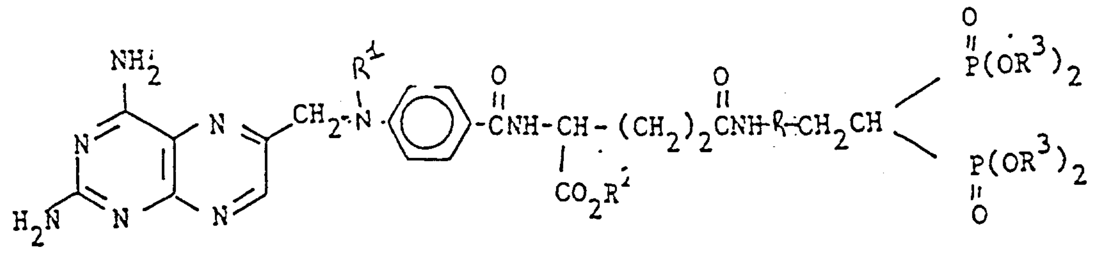

pour la mise en oeuvre du procédé selon l'une des revendications 1 à 3 caractérisé en ce qu'il consiste à réaliser une substitution nucléophile en présence de solvant sur un composé de formule :

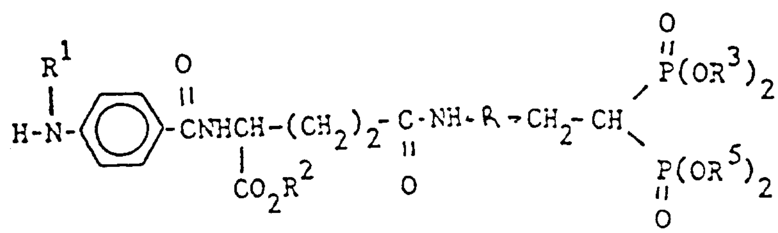

avec le 6-bromométhyl-2-4-diaminoptéridine.

**6.** Procédé de préparation d'un intermédiaire de formule :

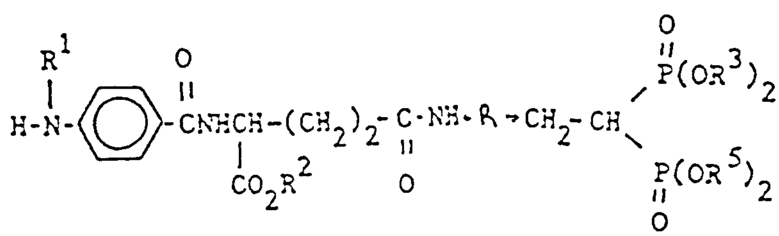

pour la mise en oeuvre du procédé selon la revendication 5 caractérisé en ce qu'il consiste à réaliser une condensation peptidique entre un synthon diphosphonique de formule :

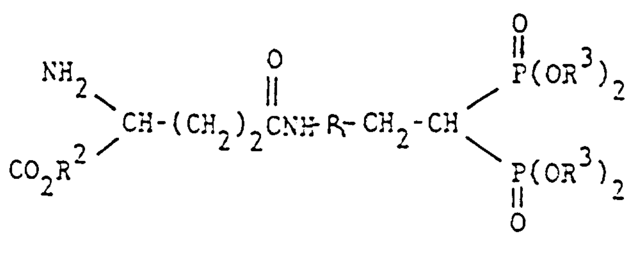

et un acide p-N-alkylaminobenzoïque.

**7.** Procédé d'obtention d'un synthon de formule :

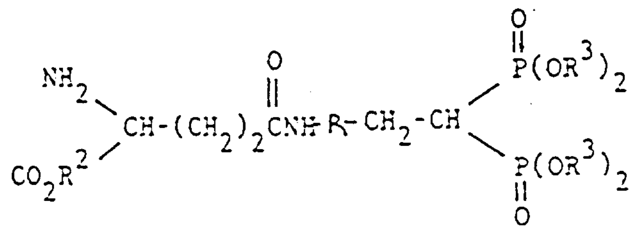

pour la mise en oeuvre du procédé selon la revendication 4 ou du procédé selon la revendication 6 caractérisé en ce qu'il consiste à :

- protéger la fonction amine de l'ester $\alpha$ - benzylique de l'acide glutamique, sous forme d'uréthane ;
- procéder au couplage peptidique dudit amino-acide avec des dérivés 2-amino-4,4-bis (dialkylphosphono) butyrate d'alkyles en présence d'un agent de couplage tel que le dicyclohexylcarbodiimide ;
- déprotéger la fonction amine par acidolyse dans l'acide trifluoracétique.

**8.** Procédé d'obtention d'un synthon de formule :

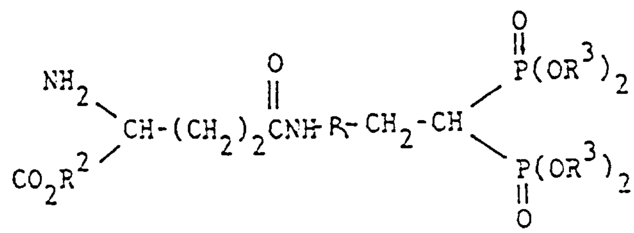

pour la mise en oeuvre du procédé selon la revendication 4 ou du procédé selon la revendication 6 caractérisé en ce qu'il consiste à réaliser une addition nucléophile de type Michaël d'un amide primaire de formule :

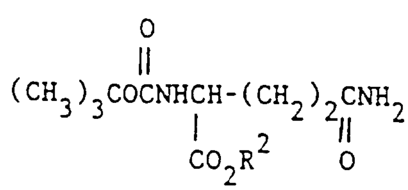

sur le vinylidène disphosphonate de tétraalkyle de formule :

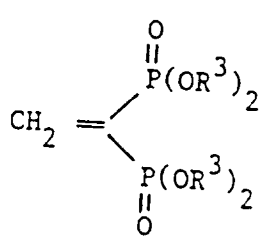

**Revendications pour l'Etat contractant suivant : GR**

1. Procédé de préparation de composés gem-diphosphoniques d'améthoptéine (méthotrexate) et de déaza N-10 améthoptérine se présentant sous la forme d'une prodrogue d'améthoptérine dont le groupement gem-diphosphonique est relié au groupement ptéroylglutamique par une liaison peptidique et répondant à la formule :

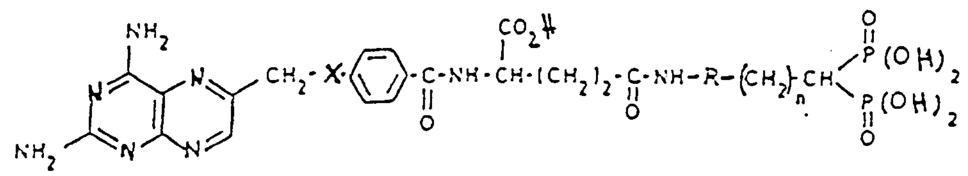

caractérisé en ce qu'il comprend les étapes consistant à faire agir sur un produit intermédiaire :

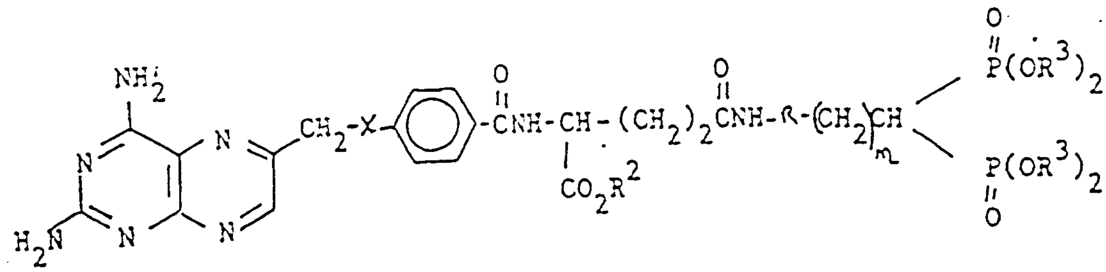

premièrement, du bromure de triméthylsilyle suivie d'une méthanolyse, pour hydrolyser les esters phosphoniques,
deuxièmement, de la soude, pour hydrolyser les esters carboxyliques,
avec dans les formules
n ≧ 0, préférentiellement n = 0, 1, 2, 3
R étant soit absent, soit constitué par un groupement

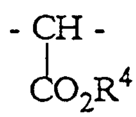

X =

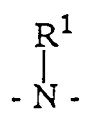 ou 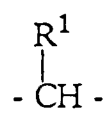

$R^1$, $R^3$, $R^4$, $R^5$ = H, ou alkyles inférieurs ($C_1$ à $C_6$)
$R^2$ = H, ou alkyles inférieurs ($C_1$ à $C_6$) et benzyle quand R présent.

2. Procédé de préparation de composés gem-diphosphoniques d'améthoptérine (méthotrexate) et de déaza N-10 améthoptérine se présentant sous la forme d'une prodrogue d'améthoptérine dont le groupement gem-diphosphonique est relié au groupement ptéroylglutamique par une liaison peptidique et répondant à la formule :

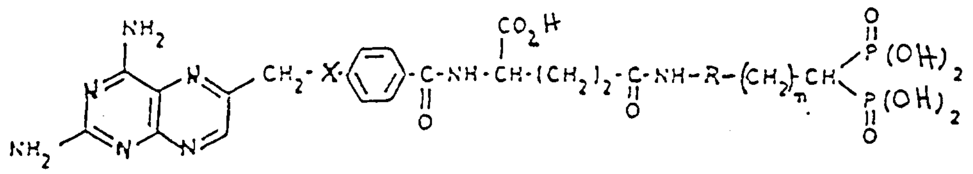

caractérisé en ce qu'il comprend les étapes consistant à faire agir sur un produit intermédiaire de formule :

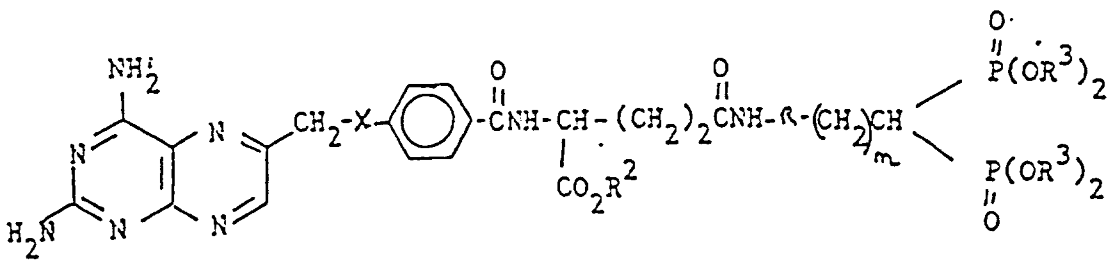

premièrement, de l'acide bromhydrique en présence d'acide acétique pour hydrolyser les esters phosphoniques ;
deuxièmement, de la soude, pour hydrolyser les esters carboxyliques,
avec dans les formules
n ≧ 0, préférentiellement n = 0, 1, 2, 3
R étant soit absent, soit constitué par un groupement

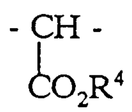

X =

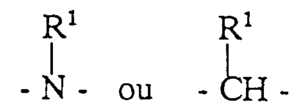

$R^1$, $R^3$, $R^4$, $R^5$ = H, ou alkyles inférieurs ($C_1$ à $C_6$)
$R^2$ = H, ou alkyles inférieurs ($C_1$ à $C_6$) et benzyle quand R présent ;

**3.** Procédé selon la revendication 1 ou 2 dans lequel
X signifie

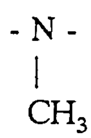

ou - $CH_2$ -

**4.** Composés gem-diphosphoniques utilisables notamment comme produits intermédiaires, de formule :

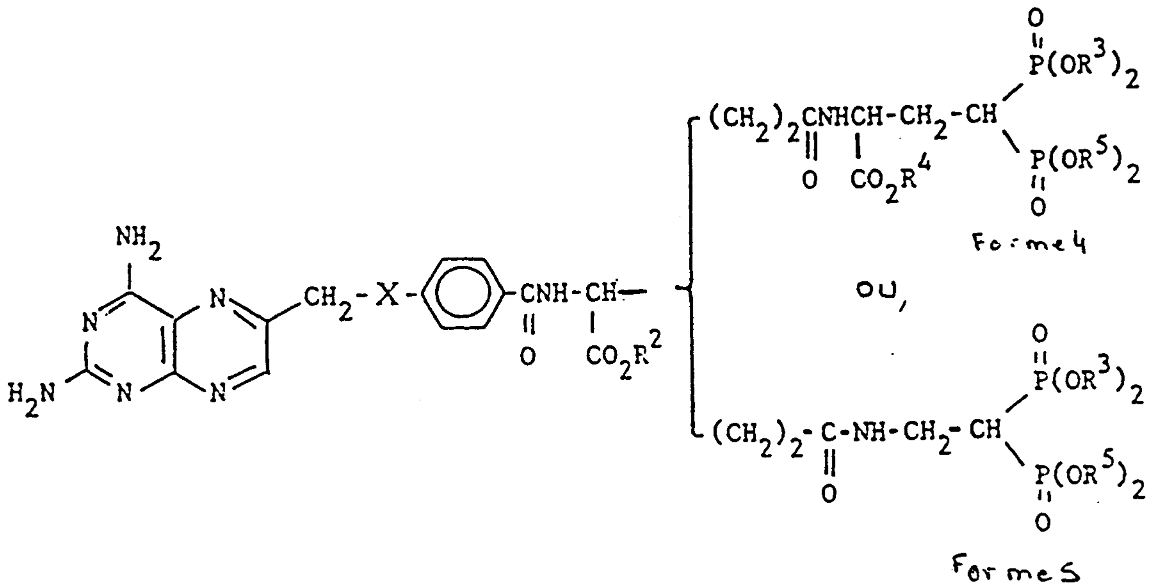

Forme 4

ou,

Forme 5

avec : X =

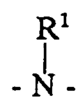 ou 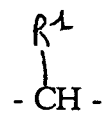

$R^1$ = H, ou alkyles inférieurs ($C_1$ à $C_6$)
$R^2$ = alkyles inférieurs ($C_1$ à $C_6$) pour la forme 5 ; benzyle pour la forme 4 ;
$R^3$, $R^4$, $R^5$ : alkyles inférieurs ($C_1$ à $C_6$) ;
ainsi que leurs acides et sels dérivés.

**5.** Composés gem-diphosphoniques, utilisables notamment comme produits intermédiaires, de formule :

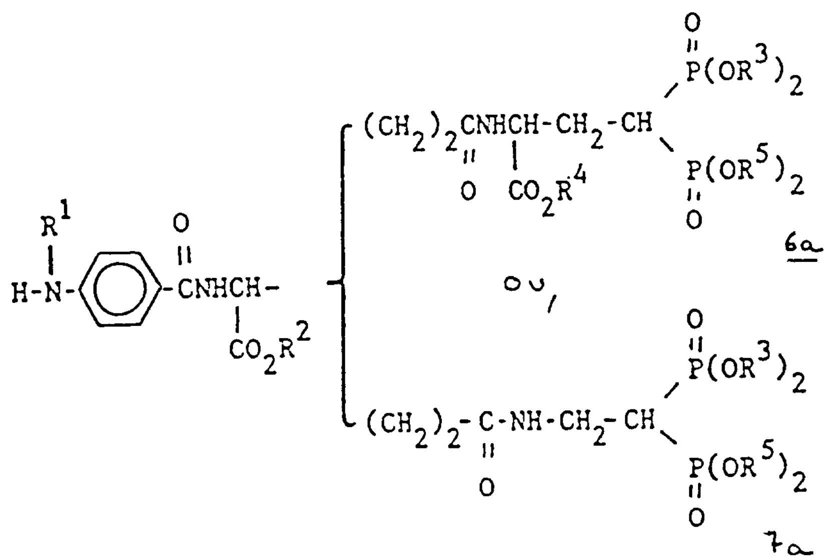

6a

ou,

7a

avec : $R^1$ = H, ou alkyles inférieurs ($C_1$ à $C_6$)
$R^3$, $R^4$, $R^5$ = alkyles inférieurs ($C_1$ à $C_6$)
$R^2$ = alkyles inférieurs ($C_1$ à $C_6$) pour 7a, benzyle pour 6a ;
ainsi que leurs acides et sels dérivés.

**6.** Composés gem-diphosphoniques, utilisables notamment comme produits intermédiaires, de formule :

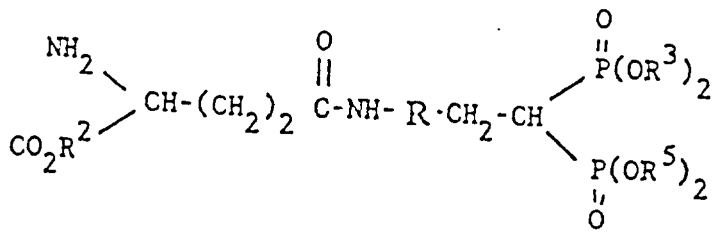

avec : R = absent ou

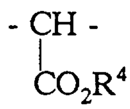

$R^2$ = H, ou alkyles inférieurs ($C_1$ à $C_6$) ; benzyle quand R présent
$R^3$, $R^4$, $R^5$ : alkyles inférieurs ($C_1$ à $C_6$) ;
ainsi que leurs acides et sels dérivés.

**Claims**

**Claims for the following Contracting States : AT, BE, CH, DE, DK, FR, GB, IT, LI, LU, NL, SE**

**1.** Gem-diphosphonic compounds of amethopterine (methotrexate) and of deaza N-10 amethopterine characterized in that they are in the form of a prodrug of amethopterine in which the gem-diphosphonic group is attached to a pteroylglutamic group by a peptide bond, and corresponding to the formula :

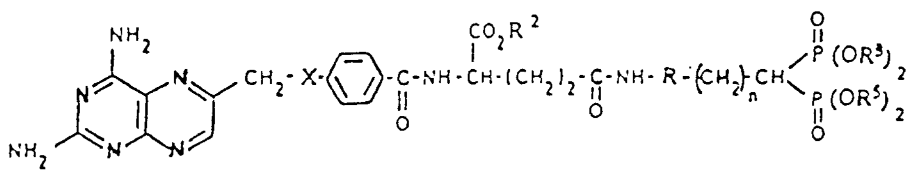

with : n ≧ 0, preferably n = 0, 1, 2, 3
R is either absent or consists of a

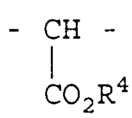

group
X =

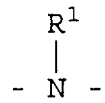 or 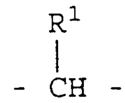

$R^1$, $R^3$, $R^4$, $R^5$ = H or lower alkyls ($C_1$ to $C_6$)
$R^2$ = H or lower alkyls ($C_1$ to $C_6$) and benzyl when R is present ;
as well as the salts and ester derivatives of the said compounds.

**2.** Compounds according to Claim 1 in which X signifies

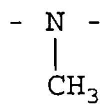

or - $CH_2$ -

**3.** Pharmaceutical composition characterized in that it contains as the active ingredient, at least one compound according to either of Claims 1 or 2.

**4.** Pharmaceutical composition according to Claim 3 which may be used in cancerology, in particular for the treatment of tumours or metastases particularly of the bones, and/or rheumatology, particularly for the treatment of intractable psoriasic rheumatisms, polymyosites, Reiter's disease and rheumatoid polyarthrites.

**5.** Gem-diphosphonic compounds which may be used particularly as intermediate compounds, of formula :

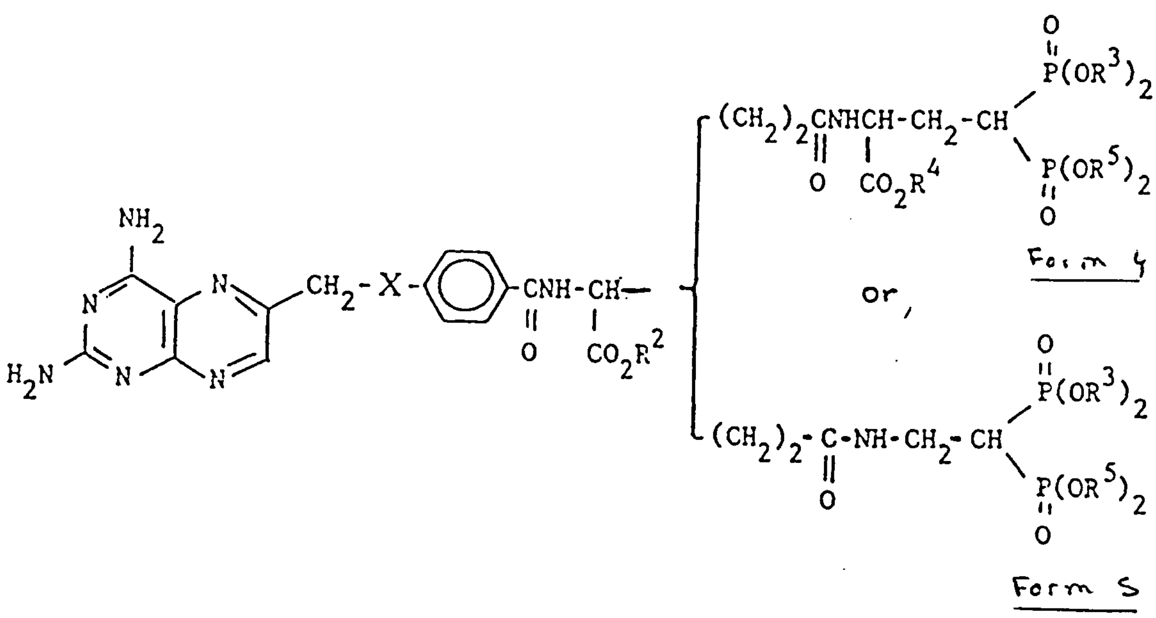

Form 4

or,

Form 5

with : X =

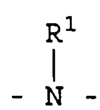 or 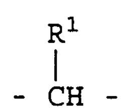

$R^1$ = H, or lower alkyls ($C_1$ to $C_6$) ;
$R^2$ = lower alkyls ($C_1$ to $C_2$) for form 5; benzyl for form 4 :
$R^3$, $R^4$, $R^5$ = lower alkyls ($C_1$ to $C_6$);
as well as their acids and salt derivatives.

**6.** Gem-diphosphonic compounds, which may be used particularly as intermediate compounds, of formula ;

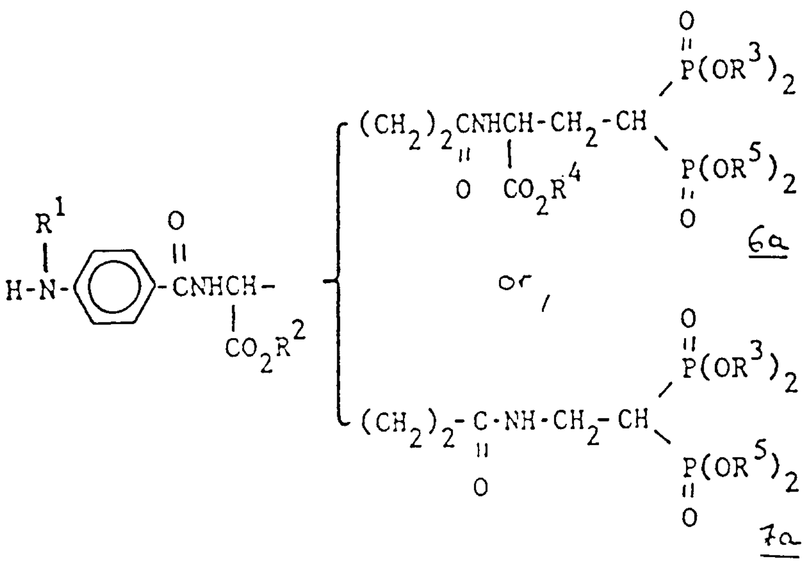

6a

or

7a

with : $R^1$ = H, or lower alkyls ($C_1$ to $C_6$)
$R^3$, $R^4$, $R^5$ = lower alkyls ($C_1$ to $C_6$) ;
$R^2$ = lower alkyls ($C_1$ to $C_2$) for form 7a; benzyl for form 6a :
as well as their acids and salt derivatives.

**7.** Gem-diphosphonic compounds, which may be used particularly as intermediate compounds, of formula :

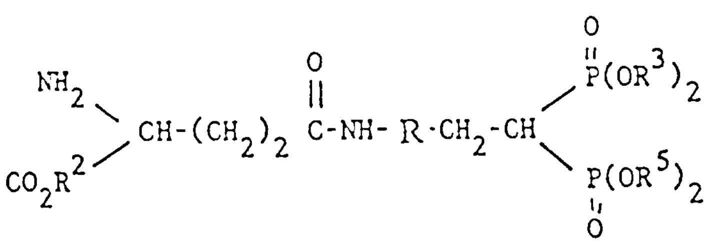

with : R is absent or

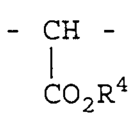

$R^2$ = H or lower alkyls ($C_1$ to $C_6$) and benzyl when R is present ;
$R^3$, $R^4$, $R^5$ = lower alkyls ($C_1$ to $C_6$) ;
as well as their acids and salt derivatives.

**8.** Method for obtaining a compound according to Claim 7, of formula :

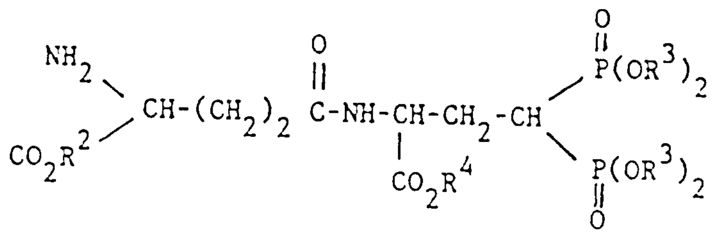

characterized in that it consists of :

- protecting the amine function of the α-benzylic ester of glutamic acid, in the form of a urethane ;
- performing a peptide coupling of the said amino acid with alkyl 2-amino-4,4-bis-(dialkylphosphono)butyrate derivatives in the presence of a coupling agent such as dicyclohexylcarbodiimide ;
- deprotecting the amine function by acidolysis in trifluoroacetic acid.

**9.** Method for obtaining a compound according to Claim 7 of formula :

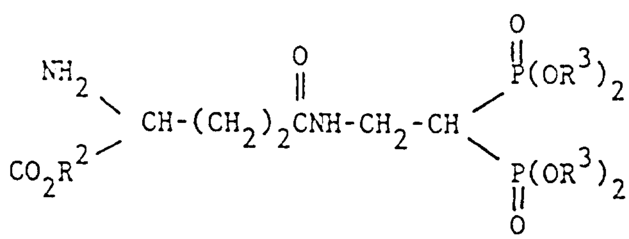

characterized in that it consists of performing a nucleophilic addition of the Michael type of a primary amide of formula :

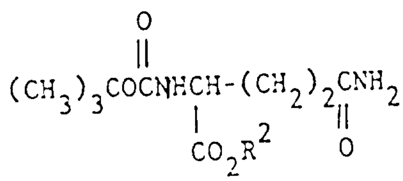

on the tetraalkyl vinylidene diphosphonate of formula :

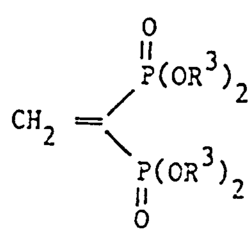

**10.** Process for obtaining a compound according to Claim 5 of formula :

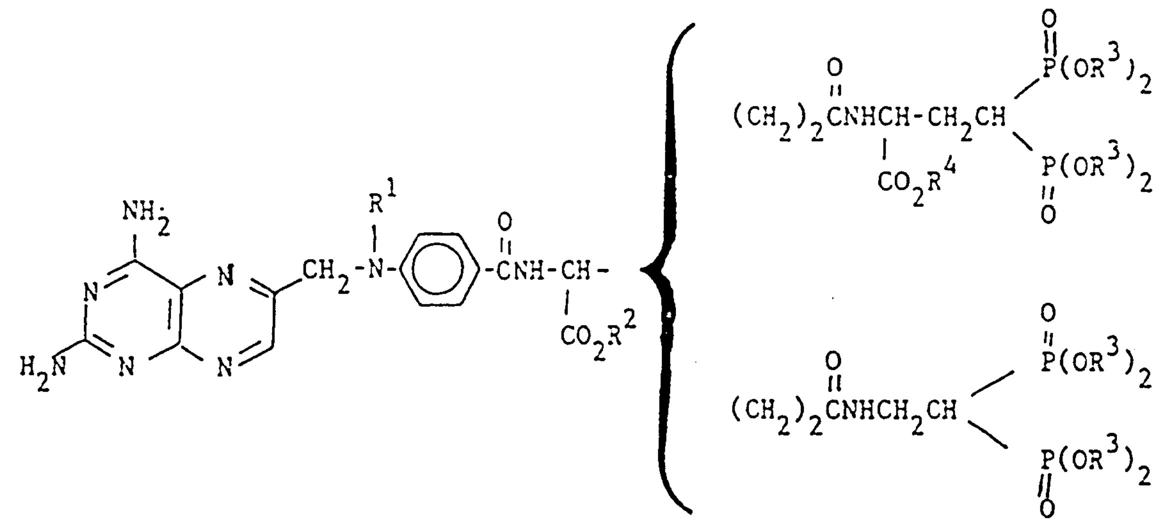

or of formula :

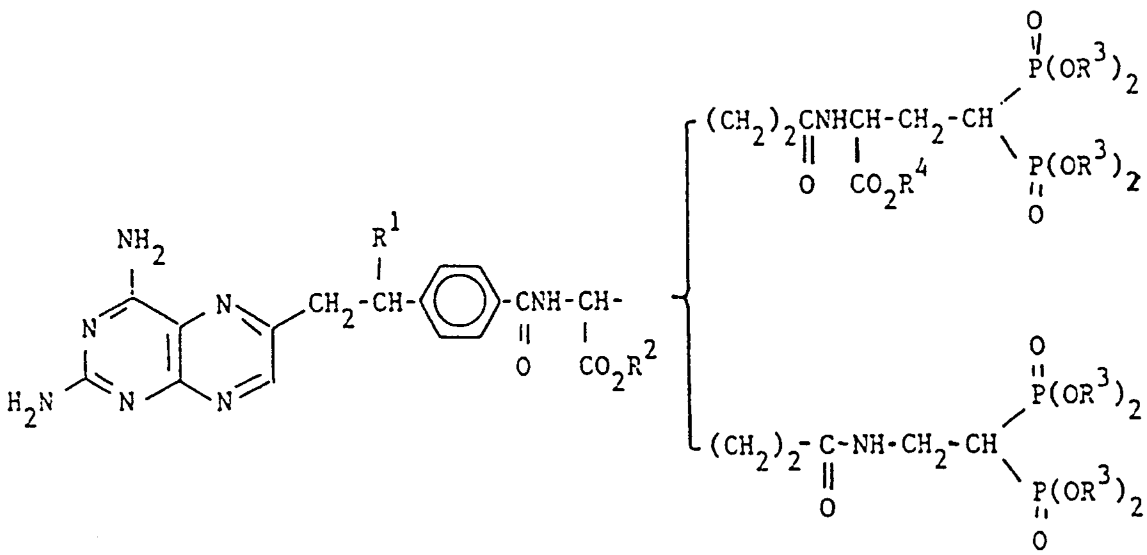

characterized in that it consists of performing a peptide condensation between the diphosphonic synthons of Claim 7 and 4-amino-4-deoxy-aza-pteroic acid or its alkyl derivatives in the 10 position; or 4-amino-4-deoxy-deaza-N-10-pteroic acid or its alkyl derivatives in the 10 position respectively.

**11.** Method for obtaining a compound according to Claim 5 of formula :

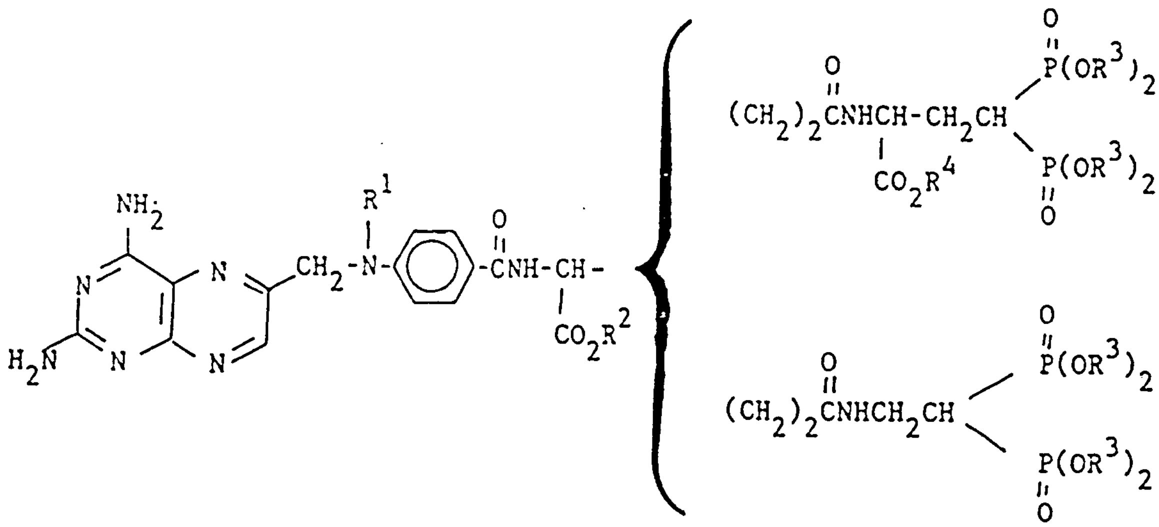

characterized in that it consists of performing a nucleophilic substitution in the presence of solvent on a compound according to Claim 6 with 6-bromomethyl-2,4-diaminopteridine.

**12.** Method for obtaining a compound according to Claim 2, characterized in that it consists of obtaining at least one of the intermediate products of Claims 5 to 7 or using at least one of the methods of Claims 8 to 11.

**13.** Use of a gem-diphosphonic compound (methotrexate) or deaza-N-10-amethopterine for producing a drug which may be used as an immunosuppressor or anti-inflammatory agent.

**Claims for the following Contracting State : ES**

**1.** Method for the preparation of gem-diphosphonic compounds of amethopterine (methotrexate) and of deaza N-10 amethopterine in the form of a prodrug of amethopterine in which the gem-diphosphonic group is attached to a pteroylglutamic group by a peptide bond, and corresponding to the formula :

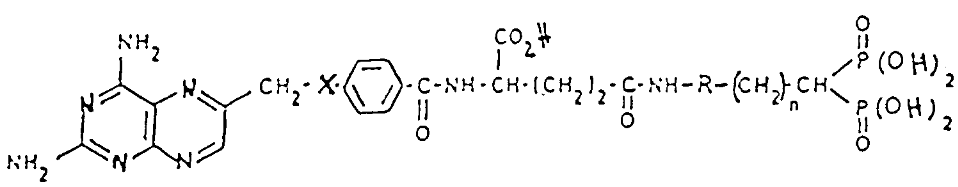

characterized in that it comprises stages consisting of reacting an intermediate product :

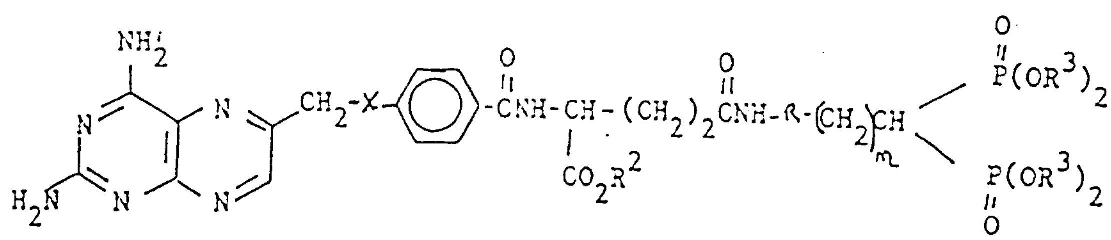

first of all with trimethylsilyl bromide, followed by methanolysis, in order to hydrolyse the phosphonic

esters, and secondly with sodium hydroxide, to hydrolyse the carboxylic esters,
with, in the formulae,
n ≧ 0, preferably n = 0, 1, 2, 3
R is either absent or consists of a

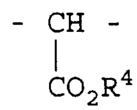

group
X =

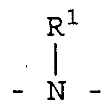 or 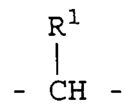

$R^1$, $R^3$, $R^4$, $R^5$ = H or lower alkyls ($C_1$ to $C_6$)
$R^2$ = H or lower alkyls ($C_1$ to $C_6$) and benzyl when R is present.

**2.** Method for the preparation of gem-diphosphonic compounds of amethopterine (methotrexate) and of deaza N-10 amethopterine in the form of a prodrug of amethopterine in which the gem-diphosphonic group is attached to a pteroylglutamic group by a peptide bond, and corresponding to the formula :

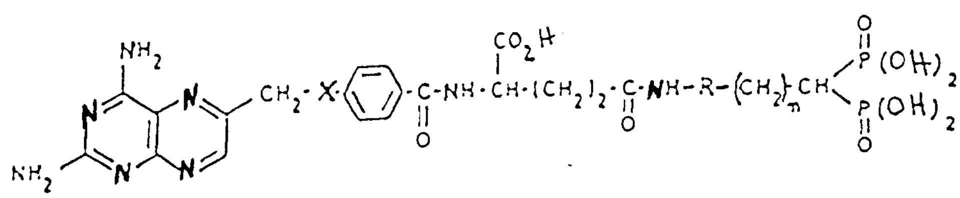

characterized in that it comprises two stages consisting of reacting an intermediate compound of formula :

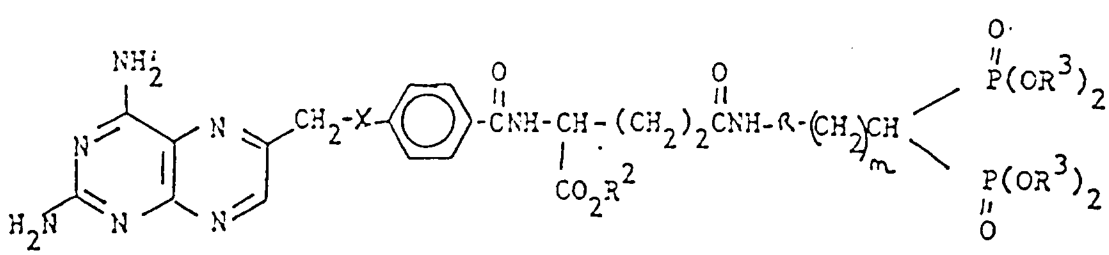

first of all with hydrobromic acid in the presence of acetic acid in order to hydrolyse the phosphonic esters ; and secondly, with sodium hydroxide to hydrolyse the carboxylic esters,
with, in the formulae
n ≧ 0, preferably n = 0, 1, 2, 3
R is either absent or consists of a

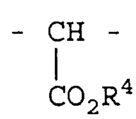

group
X =

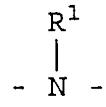 or 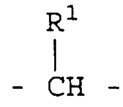

$R^1$, $R^3$, $R^4$, $R^5$ = H or lower alkyls ($C_1$ to $C_6$)
$R^2$ = H or lower alkyls ($C_1$ to $C_6$) and benzyl when R is present.

**3.** Method according to Claim 1 or 2 in which
X signifies

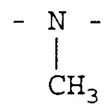

or - $CH_2$ -

**4.** Method for the preparation of an intermediate of formula :

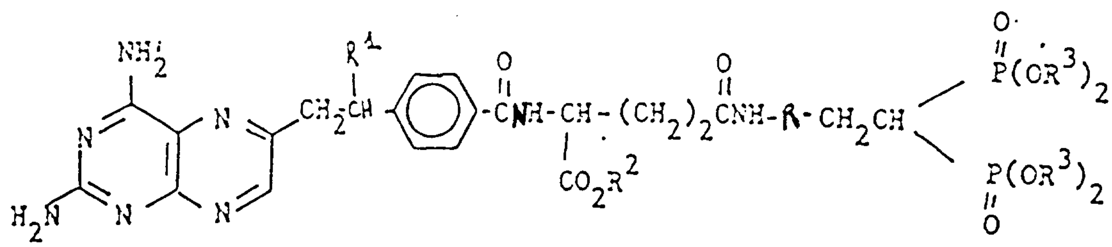

for the application of the method according to one of Claims 1, 2 or 3
characterized in that it consists of performing a peptide condensation between a diphosphonic synthon of formula :

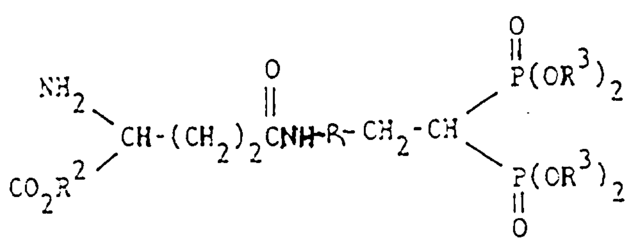

and 4-amino-4-deoxy-deaza-N-pteroic acid or its alkyl derivatives in the 10 position.

**5.** Method for the preparation of an intermediate of formula :

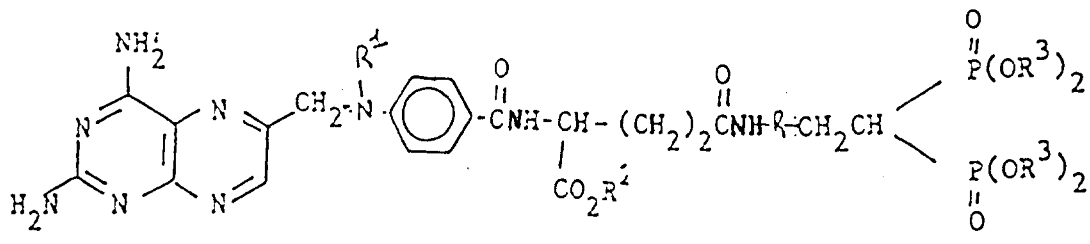

for the application of the process according to one of the Claims 1 to 3 characterized in that it consists of performing a nucleophilic substitution in the presence of solvent on a compound of formula :

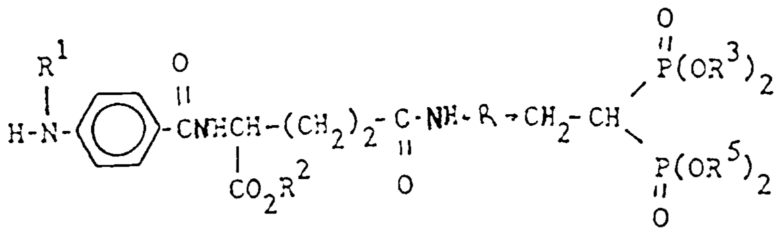

with 6-bromomethyl-2,4-diaminopteridine.

**6.** Method for the preparation of an intermediate of formula :

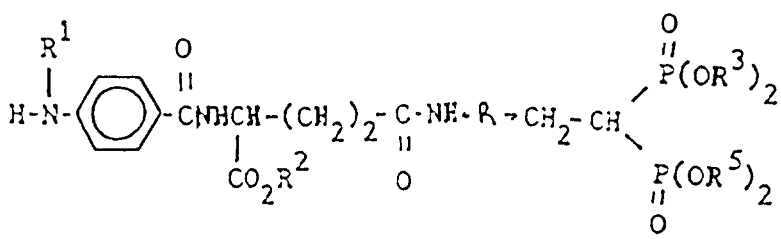

for the application of the method according to Claim 5 characterized in that it consists of performing a peptide condensation between a diphosphonic synthon of formula :

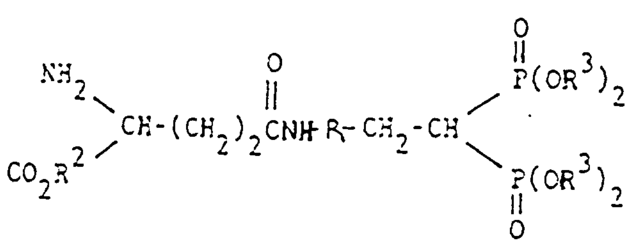

and a p-N-alkylaminobenzoic acid.

7. Method for preparing a synthon of formula :

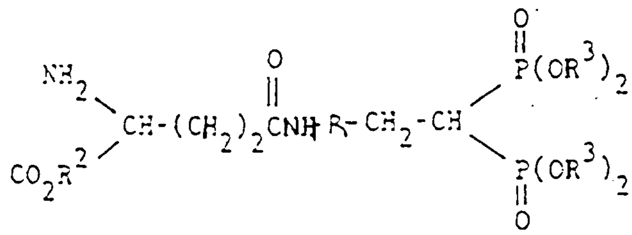

for the application of the method according to Claim 4 or the method according to Claim 6 characterized in that it consists of :

- protecting the amine function of the α-benzylic ester of glutamic acid, in the form of a urethane ;
- performing a peptide coupling of the said amino acid with alkyl 2-amino-4,4-bis-(dialkylphosphono)butyrate derivatives in the presence of a coupling agent such as dicyclohexylcarbodiimide ;
- deprotecting the amine function by acidolysis in trifluoroacetic acid.

8. Method for preparing a synthon of formula :

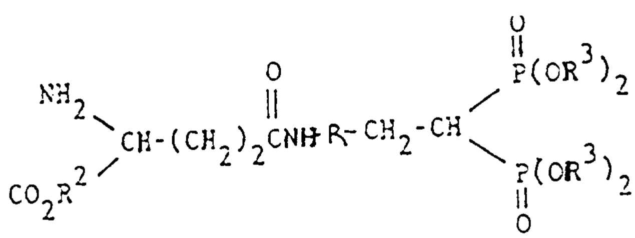

for the application of the method according to Claim 4 or the method according to Claim 6 characterized in that it consists of performing a nucleophilic addition of the Michael type of a primary amide of formula :

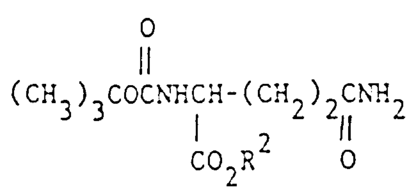

on the tetraalkyl vinylidene diphosphonate of formula :

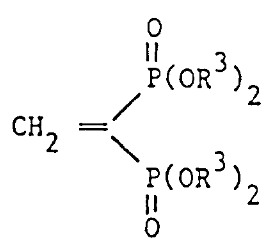

**Claims for the following Contracting State : GR**

1. Method for the preparation of gem-diphosphonic compounds of amethopterine (methotrexate) and of deaza N-10 amethopterine in the form of a prodrug of amethopterine in which the gem-diphosphonic group is attached to a pteroylglutamic group by a peptide bond, and corresponding to the formula :

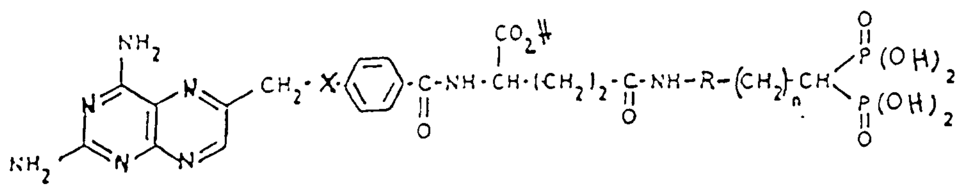

characterized in that it comprises stages consisting of reacting an intermediate product :

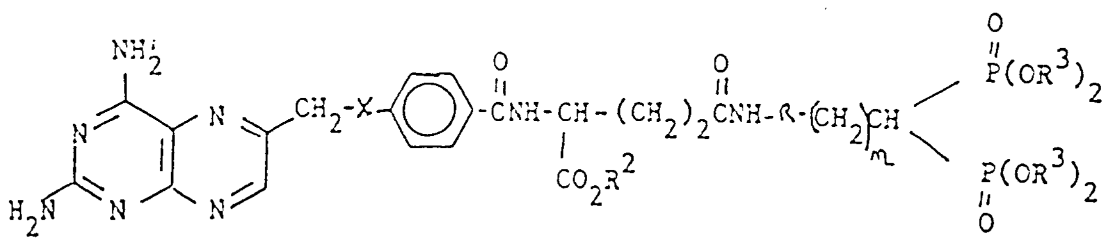

first of all with trimethylsilyl bromide, followed by methanolysis, in order to hydrolyse the phosphonic esters,
and secondly with sodium hydroxide, to hydrolyse the carboxylic esters,
with, in the formulae,
$n \geqq 0$, preferably n = 0, 1, 2, 3
R is either absent or consists of a

$$\begin{array}{c} -CH- \\ | \\ CO_2R^4 \end{array}$$

group
X =

$$\begin{array}{c} R^1 \\ | \\ -\ N- \end{array} \quad \text{or} \quad \begin{array}{c} R^1 \\ | \\ -\ CH- \end{array}$$

$R^1$, $R^3$, $R^4$, $R^5$ = H or lower alkyls ($C_1$ to $C_6$)
$R^2$ = H or lower alkyls ($C_1$ to $C_6$) and benzyl when R is present.

2. Method for the preparation of gem-diphosphonic compounds of amethopterine (methotrexate) and of deaza N-10 amethopterine in the form of a prodrug of amethopterine in which the gem-diphosphonic group is attached to a pteroylglutamic group by a peptide bond, and corresponding to the formula :

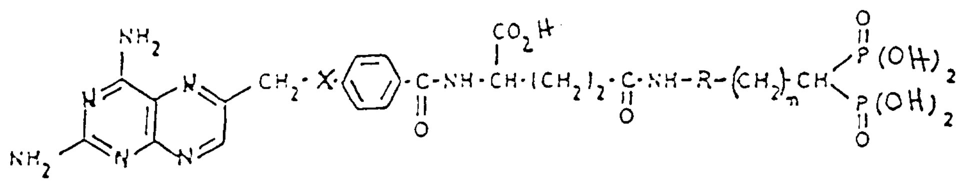

characterized in that it comprises two stages consisting of reacting an intermediate compound of formula :

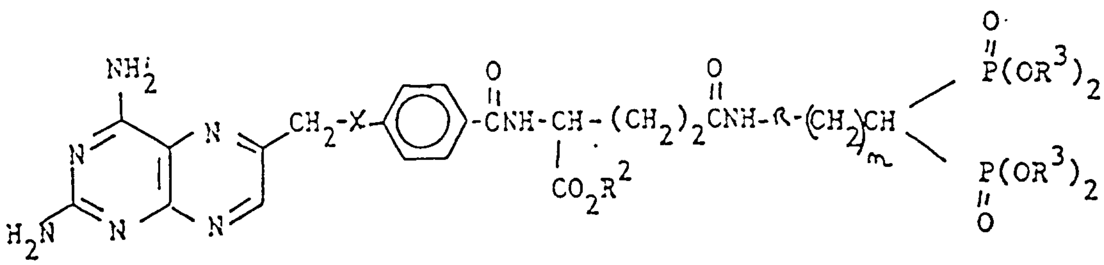

first of all with hydrobromic acid in the presence of acetic acid in order to hydrolyse the phosphonic esters ;
and secondly, with sodium hydroxide to hydrolyse the carboxylic esters,
with, in the formulae
n ≧ 0, preferably n = 0, 1, 2, 3
R is either absent or consists of a

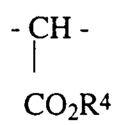

group X =

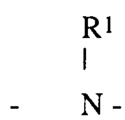

or

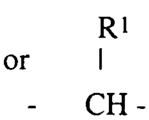

$R^1$, $R^3$, $R^4$, $R^5$ = H or lower alkyls ($C_1$ to $C_6$)
$R^2$ = H or lower alkyls ($C_1$ to $C_6$) and benzyl when R is present.

**3.** Method according to Claim 1 or 2 in which
X signifies

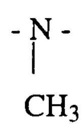

or - $CH_2$ -

**4.** Gem-diphosphonic compounds which may be used particularly as intermediate compounds, of formula :

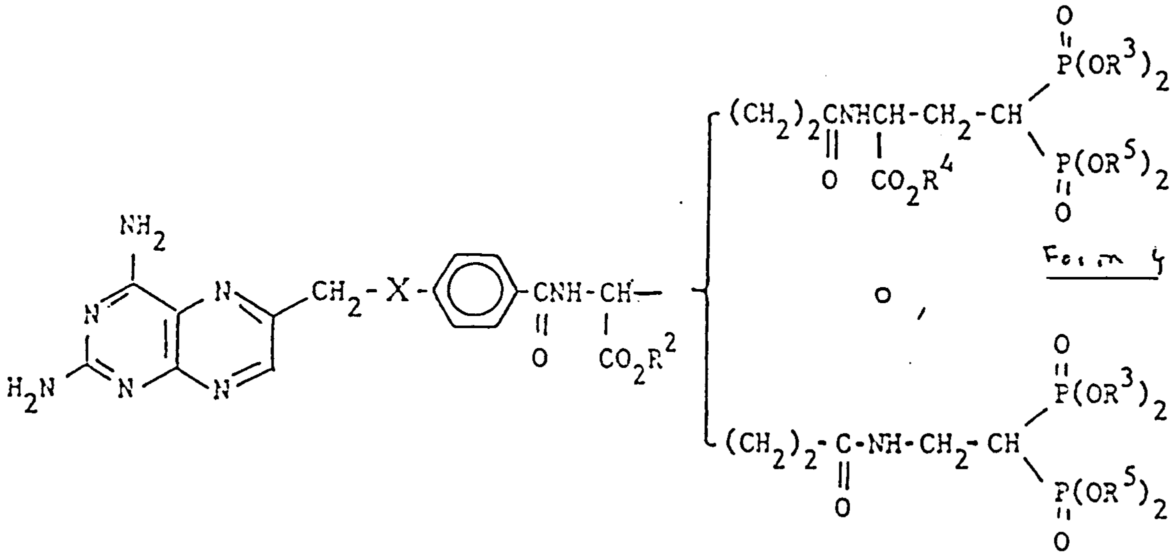

Form 4

Form 5

with: X =

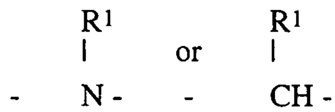

$R^1$ = H, or lower alkyls ($C_1$ to $C_6$) ;
$R^2$ = lower alkyls ($C_1$ to $C_2$) for form 5; benzyl for form 4 :
$R^3$, $R^4$, $R^5$ = lower alkyls ($C_1$ to $C_6$);
as well as their acids and salt derivatives.

**5.** Gem-diphosphonic compounds, which may be used particularly as intermediate compounds, of formula ;

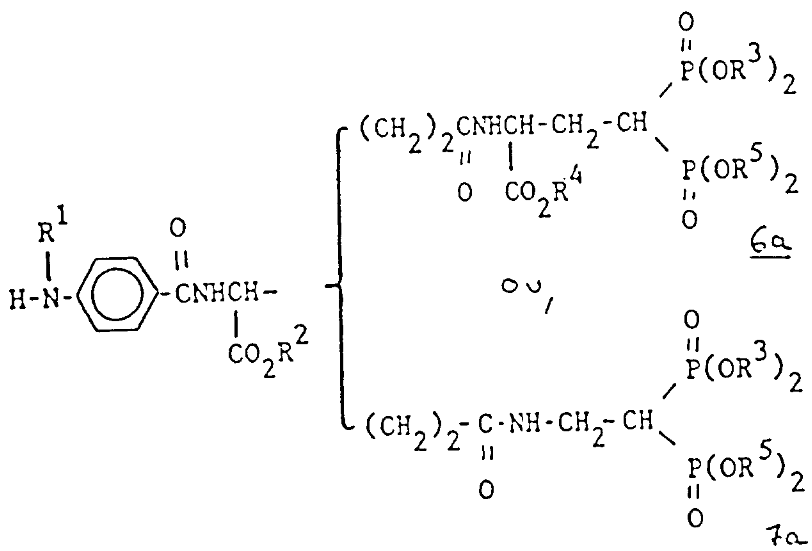

6a

7a

with : $R^1$ = H, or lower alkyls ($C_1$ to $C_6$)

$R^3$, $R^4$, $R^5$ = lower alkyls ($C_1$ to $C_6$);
$R^2$ = lower alkyls ($C_1$ to $C_2$) for form 7a; benzyl for form 6a :
as well as their acids and salt derivatives.

**6.** Gem-diphosphonic compounds, which may be used particularly as intermediate compounds, of formula :

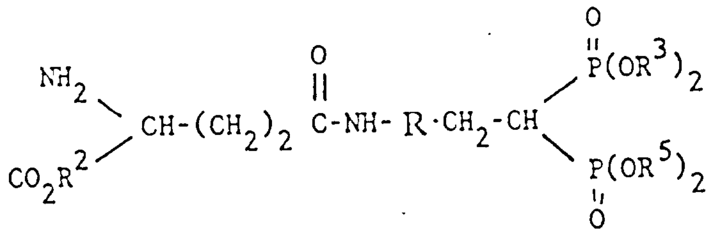

with : R is absent or

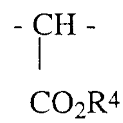

$R^2$ = H or lower alkyls ($C_1$ to $C_6$) and benzyl when R is present ;
$R^3$, $R^4$, $R^5$ = lower alkyls ($C_1$ to $C_6$;
as well as their acids and salt derivatives.

**Patentansprüche**

**Patentansprüche für folgende Vertragsstaaten : AT, BE, CH, DE, DK, FR, GB, IT, LI, LU, NL, SE**

**1.** Gem-Diphosphonsäure-Amethopterin- (Methotrexat-) und Deaza-N-10-amethopterin-Verbindungen **dadurch gekennzeichnet,**
daß sie in Form eines Amethopterin-Propharmakons vorliegen, dessen geminale Diphosphonsäure-Gruppierung über eine Peptidbindung mit der Pteroylglutaminsäure-Gruppierung verbunden ist, und welches folgende Formel aufweist:

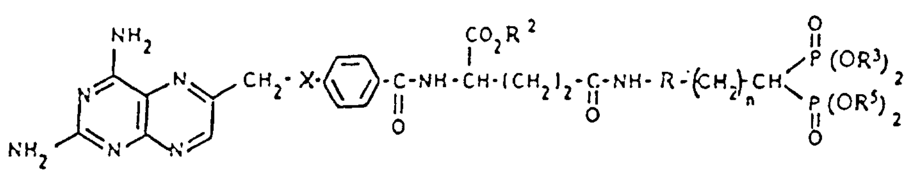

wobei n ≧ 0, bevorzugt n = 0, 1, 2, 3 bedeutet
und wobei R entweder nicht vorhanden ist oder aus der Gruppe

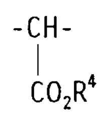

besteht,
X die Bedeutung

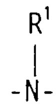 oder 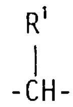

hat, und wobei
$R^1$, $R^3$, $R^4$, $R^5$ die Bedeutung H oder niedriges Alkyl ($C_1$ bis $C_6$) hat und
$R^2$ die Bedeutung H oder niedriges Alkyl ($C_1$ bis $C_6$) und Benzyl hat, wenn R vorhanden ist.

2. Verbindungen nach Anspruch 1 oder 2, wobei
X die Bedeutung

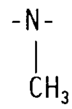

oder $-CH_2-$ hat.

3. Pharmazeutische Zusammensetzung,
**dadurch gekennzeichnet,**
daß sie als Wirkstoff mindestens eine Verbindung gemäß einem der Ansprüche 1 oder 2 enthält.

4. Pharmazeutische Zusammensetzung nach Anspruch 3, anwendbar bei der Krebsbekämpfung, insbesondere bei der Behandlung von Tumoren oder Metastasen, hauptsächlich an den Knochen, und/oder bei der Behandlung von Rheuma, insbesondere bei der Behandlung chronischer rheumatischer Psoriasis, von Polymositis, der Reiter-Krankheit und rheumatoider Polyarthritis.

5. Gem-Diphosphonsäure-Verbindungen, die insbesondere als Zwischenprodukte anzuwenden sind und die Formel

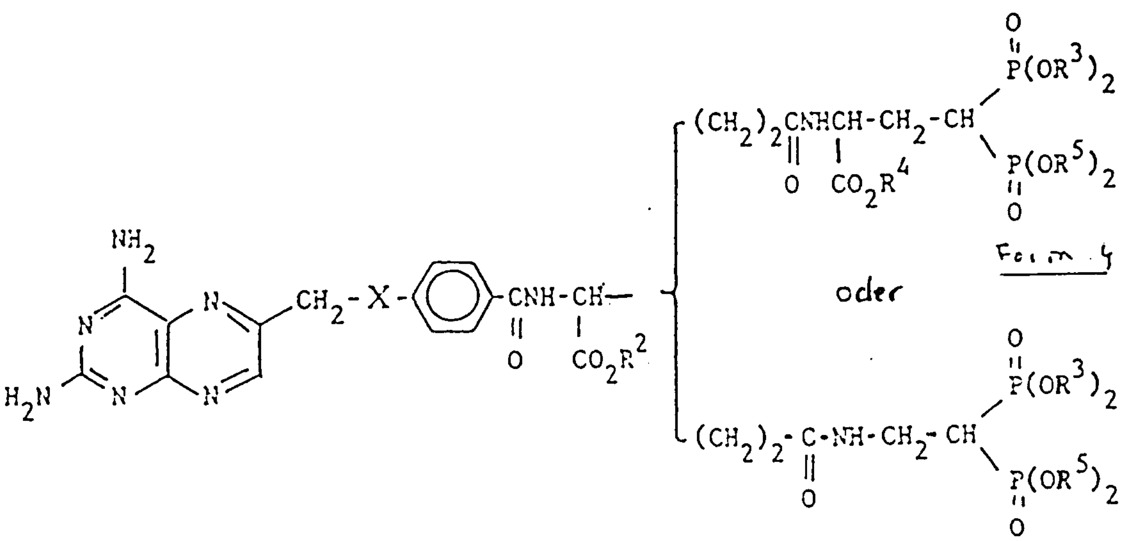

Form. 4

oder

Form. 5

aufweisen, wobei
X die Bedeutung

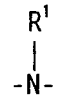 oder 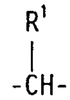

hat, und wobei
$R^1$ die Bedeutung H oder niedriges Alkyl ($C_1$ bis $C_6$) hat,
$R^2$ die Bedeutung niedriges Alkyl ($C_1$ bis $C_6$) für die Form 5; Benzyl für die Form 4 hat,
$R^3$, $R^4$, $R^5$ die Bedeutung niedriges Alkyl ($C_1$ bis $C_6$) hat,
sowie deren Säure- und Salzderivate.

**6.** Gem-Diphosphonsäure-Verbindungen, die insbesondere als Zwischenprodukte anzuwenden sind und die Formel

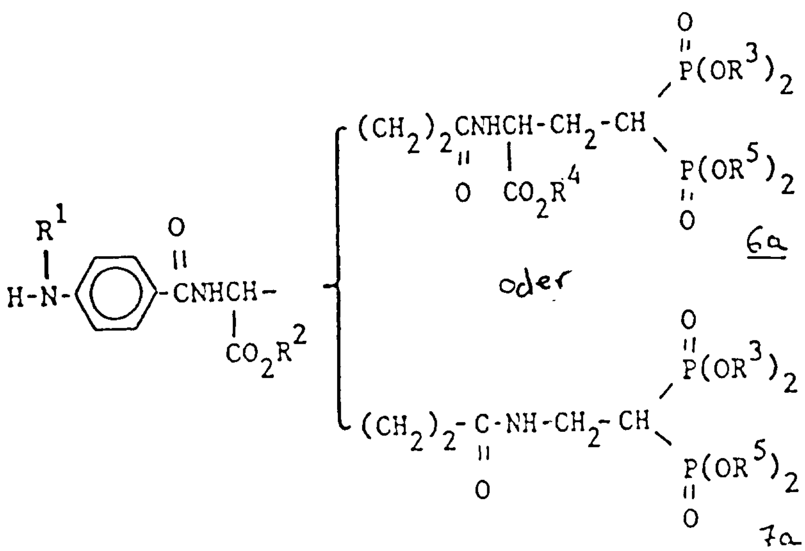

6a oder 7a

aufweisen, wobei
$R^1$ die Bedeutung H oder niedriges Alkyl ($C_1$ bis $C_6$) hat,
$R^3$, $R^4$, $R^5$ die Bedeutung niedriges Alkyl ($C_1$ bis $C_6$) hat,
$R^2$ die Bedeutung niedriges Alkyl ($C_1$ bis $C_6$) für 7a, Benzyl für 6a hat,
sowie deren Säure- und Salzderivate.

**7.** Gem-Diphosphonsäure-Verbindungen, die insbesondere als Zwischenprodukte anzuwenden sind und die Formel

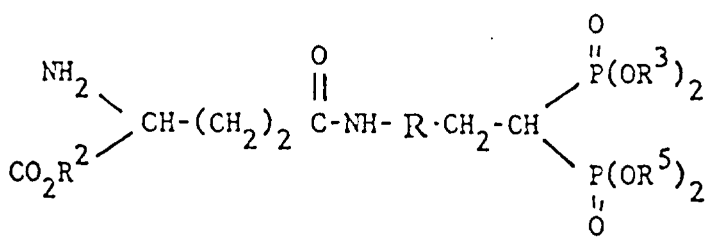

aufweisen, wobei
R nicht vorhanden ist oder

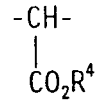

bedeutet,
$R^2$ die Bedeutung H oder niedriges Alkyl ($C_1$ bis $C_6$); Benzyl hat, wenn R vorhanden ist,
$R^3$, $R^4$, $R^5$ die Bedeutung niedriges Alkyl ($C_1$ bis $C_6$) hat,
sowie deren Säure- und Salzderivate.

**8.** Verfahren zum Erhalten einer Verbindung gemäß Anspruch 7 mit der Formel

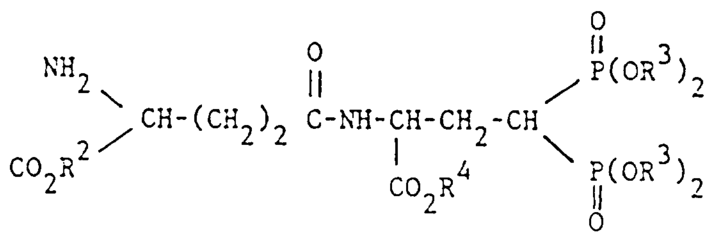

**dadurch gekennzeichnet,**
daß es besteht aus

- Schutz der Aminfunktion des $\alpha$-Benzylesters der Glutaminsäure in Form eines Urethans;
- Peptidkopplung der besagten Aminosäure mit 2-Amino-4,4-bis(dialkylphosphon)alkylbutyrat-Derivaten in Gegenwart eines Kopplungsmittels, wie z.B. Dicyclohexylcarbodiimid;
- Entfernung des Schutzes der Aminfunktion durch Säurehydrolyse mit Trifluoressigsäure.

**9.** Verfahren zum Erhalten einer Verbindung gemäß Anspruch 7 mit der Formel

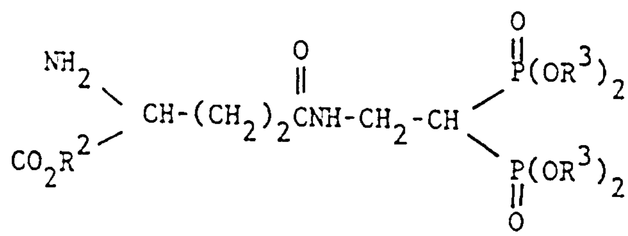

**dadurch gekennzeichnet,**
daß es in der Durchführung einer nukleophilen Michael-Addition eines Primäramids der Formel

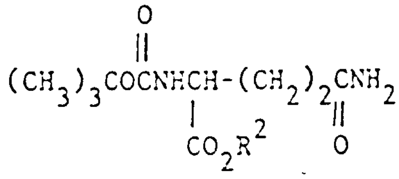

an das Tetraalkylvinylidendiphosphonat der Formel

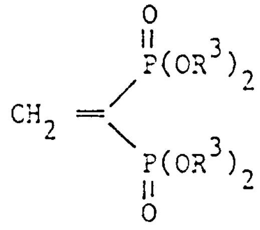

besteht.

**10.** Verfahren zum Erhalten einer Verbindung gemäß Anspruch 5 mit der Formel

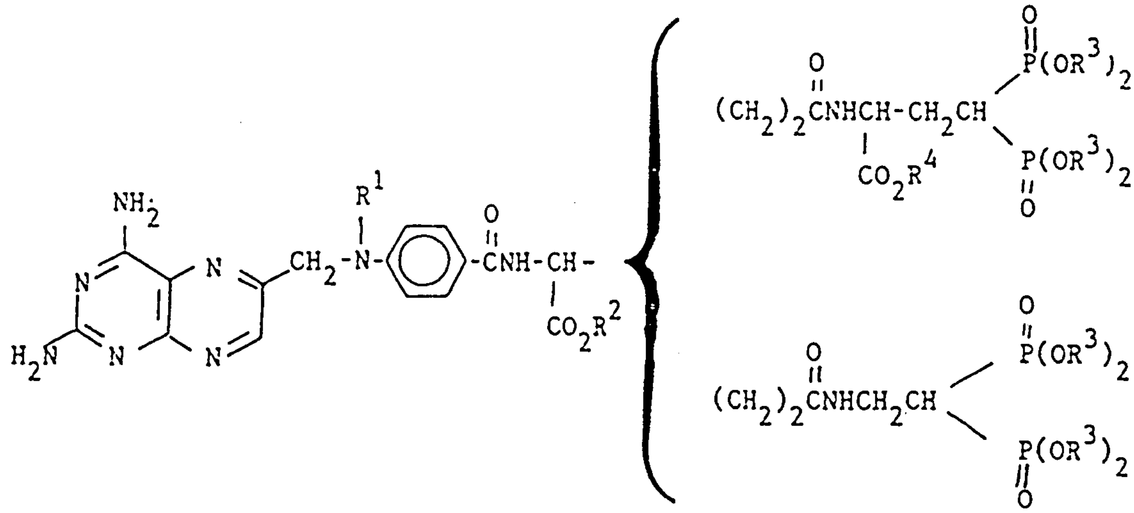

oder der Formel

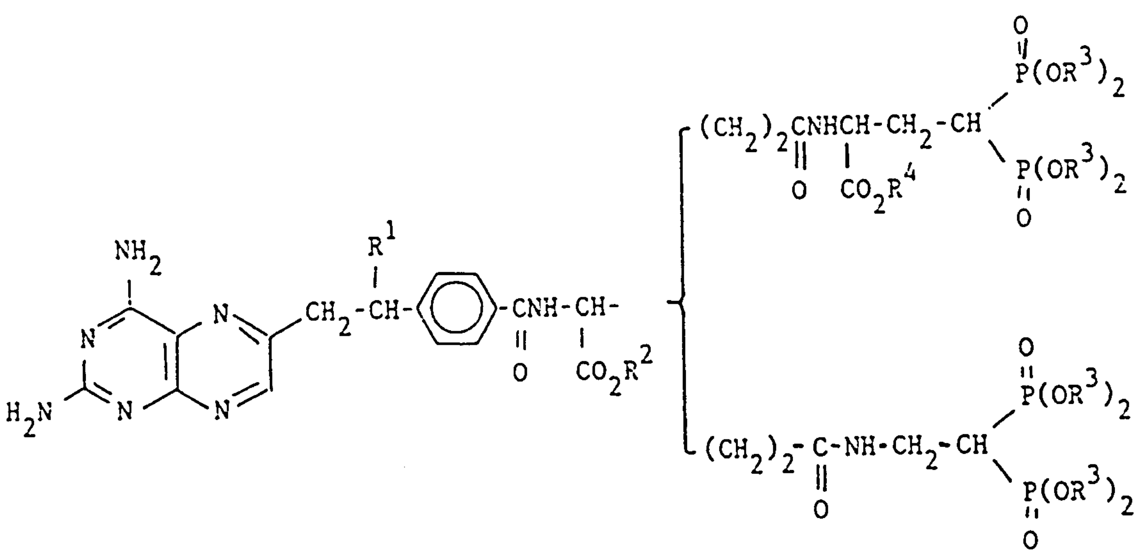

**dadurch gekennzeichnet**,
daß es in der Durchführung einer Peptidkondensation der Diphosphonsäure-Synthonegemäß Anspruch 7 mit 4-Amino-4-deoxy aza-pteroinsäure oder deren in 10-Position alkylierten Derivaten bzw. der 4-Amino-4-deoxy-deaza-N-10-pteroinsäure oder deren in 10-Position alkylierten Derivaten besteht.

**11.** Verfahren zum Erhalten einer Verbindung gemäß Anspruch 5 mit der Formel

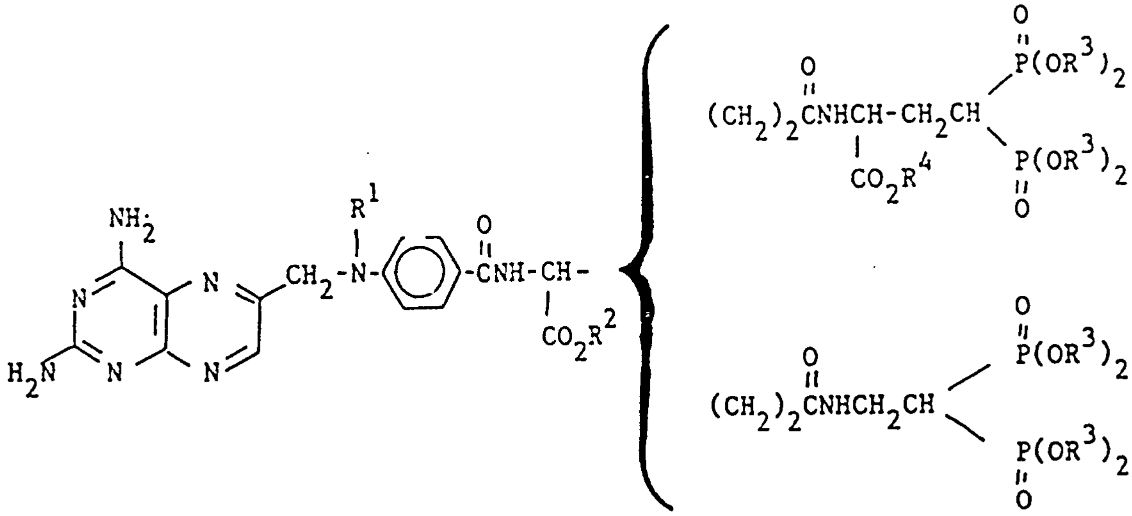

**dadurch gekennzeichnet,**
daß es in der Durchführung einer nukleophilen Substitution in Gegenwart eines Lösungsmittels an einer Verbindung gemäß Anspruch 6 mit 6-Brommethyl-2-4-diaminopteridin besteht.

**12.** Verfahren zum Erhalten einer Verbindung gemäß Anspruch 2, dadurch gekennzeichnet, daß es darin besteht, mindestens eines der Zwischenprodukte der Ansprüche 5 bis 7 durch Durchführung mindestens eines der Verfahren der Ansprüche 8 bis 11 zu erhalten.

**13.** Verwendung einer Gem-Diphosphonsäure-Amethopterin- (Methotrexat-) oder Deaza-N-10-amethopterin-Verbindung zur Herstellung eines Medikaments, das als Mittel zur Immununterdrückung oder als entzündungshemmendes Mittel eingesetzt wird.

**Patentansprüche für folgenden Vertragsstaat : ES**

**1.** Verfahren zur Herstellung von gem-Diphosphonsäure-Amethopterin- (Methotrexat-) und Deaza-N-10-amethopterin-Verbindungen in Form eines Amethopterin-Propharmakons, dessen geminale Diphosphonsäure-Gruppierung über eine Peptidbindung mit der Pteroylglutaminsäure-Gruppierung verbunden ist, und welches folgende Formel aufweist:

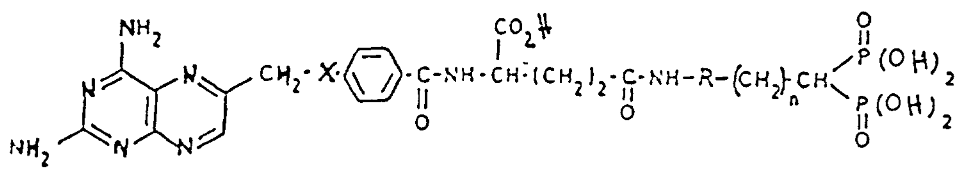

**gekennzeichnet durch** die Schritte, daß man auf ein Zwischenprodukt

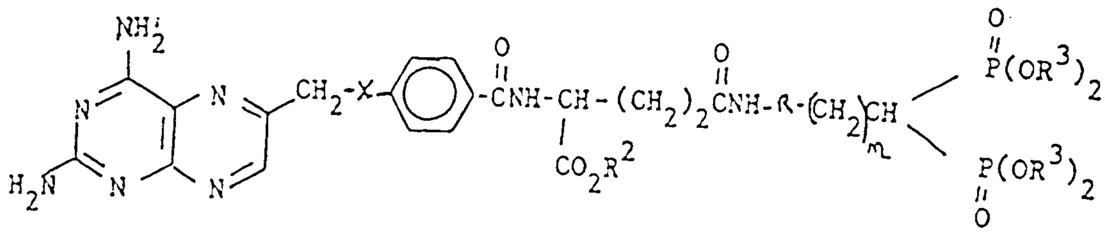

zuerst Trimethylsilylbromid einwirken läßt, gefolgt von einer Methanolyse zur Hydrolyse der Phosphonsäureester,
zweitens Soda einwirken läßt, um die Carboxylester zu hydrolysieren,
wobei in den Formeln
n ≧ 0, bevorzugt n = 0, 1, 2, 3 bedeutet
und wobei R entweder nicht vorhanden ist oder aus der Gruppe

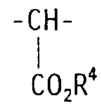

besteht,
X die Bedeutung

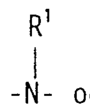 oder 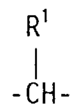

hat, und wobei
$R^1$, $R^3$, $R^4$, $R^5$ die Bedeutung H oder niedriges Alkyl ($C_1$ bis $C_6$) hat und
$R^2$ die Bedeutung H oder niedriges Alkyl ($C_1$ bis $C_6$) und Benzyl hat, wenn R vorhanden ist.

**2.** Verfahren zur Herstellung von gem-Diphosphonsäure-Amethopterin- (Methotrexat-) und Deaza-N-10-amethopterin-Verbindungen in Form eines Amethopterin-Propharmakons, dessen geminale Diphosphonsäure-Gruppierung über eine Peptidbindung mit der Pteroylglutaminsäure-Gruppierung verbunden ist, und welches folgende Formel aufweist:

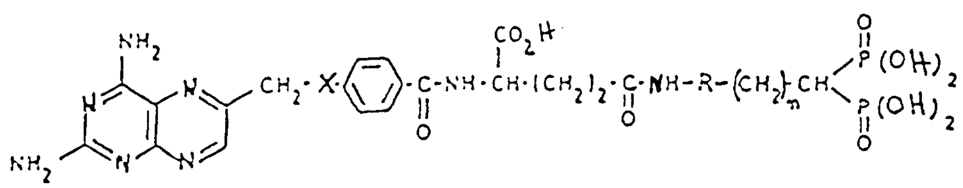

**gekennzeichnet durch** die Schritte, daß man auf ein Zwischenprodukt der Formel

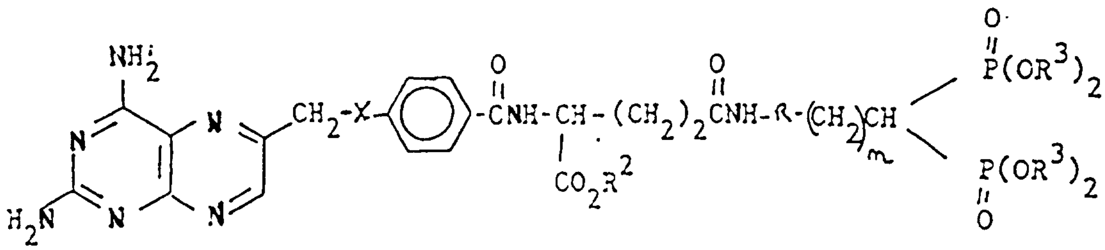

zuerst Bromwasserstoffsäure in Gegenwart von Essigsäure einwirken läßt, um die Phosphonsäureester zu hydrolysieren;
zweitens Soda einwirken läßt, um die Carboxylester zu hydrolysieren,
wobei in den Formeln
n ≧ 0, bevorzugt n = 0, 1, 2, 3 bedeutet
und wobei R entweder nicht vorhanden ist oder aus der Gruppe

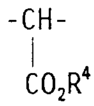

besteht,
X die Bedeutung

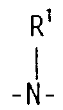 oder 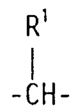

hat, und wobei
$R^1$, $R^3$, $R^4$, $R^5$ die Bedeutung H oder niedriges Alkyl ($C_1$ bis $C_6$) hat und
$R^2$ die Bedeutung H oder niedriges Alkyl ($C_1$ bis $C_6$) und Benzyl hat, wenn R vorhanden ist.

**3.** Verfahren nach Anspruch 1 oder 2, wobei
X die Bedeutung

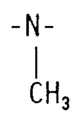

oder $-CH_2-$ hat.

**4.** Verfahren zur Herstellung eines Zwischenproduktes der Formel

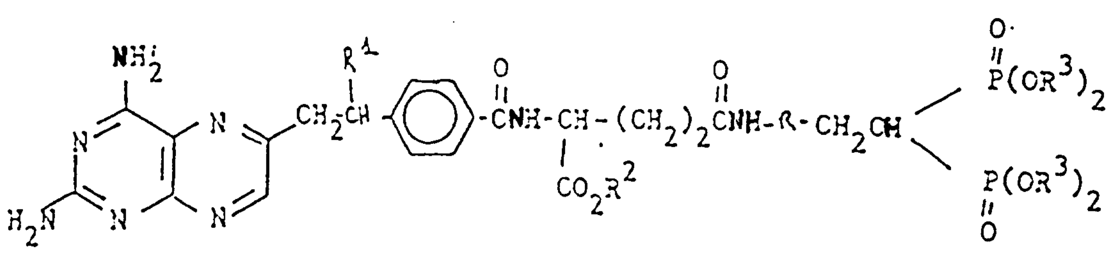

zur Durchführung des Verfahrens gemäß einem der Ansprüche 1, 2 oder 3,

**dadurch gekennzeichnet**,
daß es in der Durchführung einer Peptidkondensation eines Diphosphonsäure-Synthons der Formel

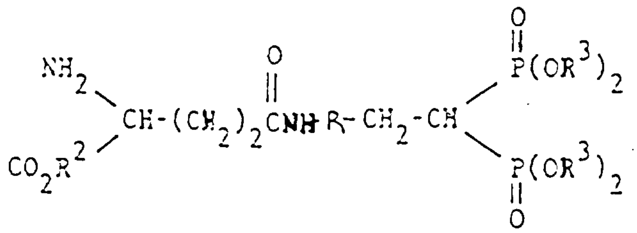

mit der 4-Amino-4-deoxy-deaza-N-pteroinsäure oder deren in 10-Position alkylierten Derivaten besteht.

**5.** Verfahren zur Herstellung eines Zwischenproduktes der Formel

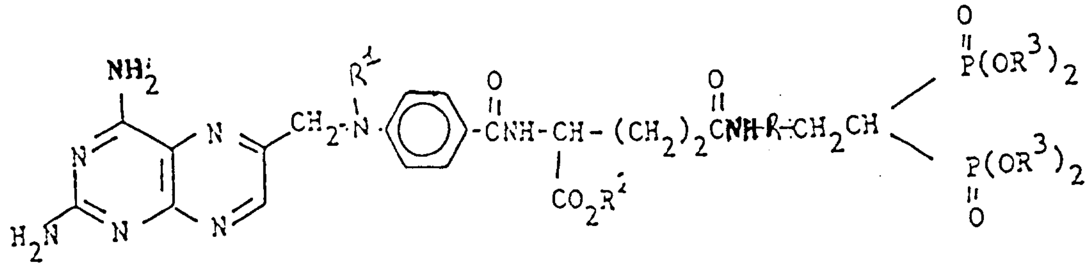

zur Durchführung des Verfahrens gemäß einem der Ansprüche 1 bis 3,
**dadurch gekennzeichnet,**
daß es in der Durchführung einer nukleophilen Substitution in Gegenwart eines Lösungsmittels an einer Verbindung der Formel

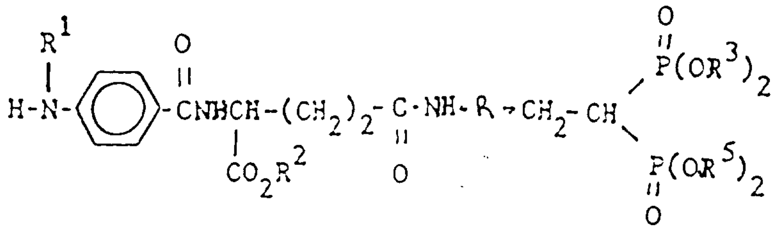

mit 6-Brommethyl-2-4-diaminopteridin besteht.

**6.** Verfahren zur Herstellung eines Zwischenproduktes der Formel

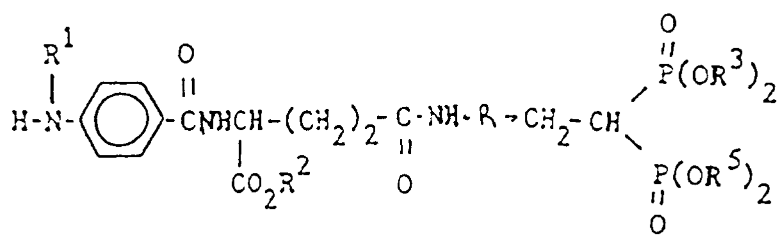

zur Durchführung des Verfahrens gemäß Anspruch 5, dadurch gekennzeichnet, daß es in der Durchführung einer Peptidkondensation eines Diphosphonsäure-Synthons der Formel

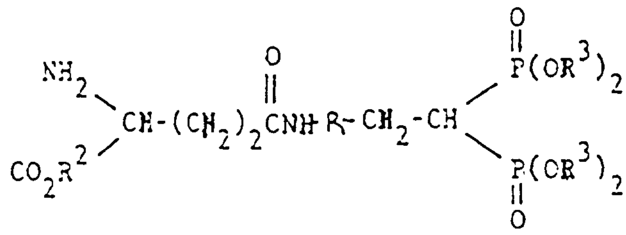

mit einer p-N-Alkylaminbenzoesäure besteht.

**7.** Verfahren zum Erhalten eines Synthons der Formel

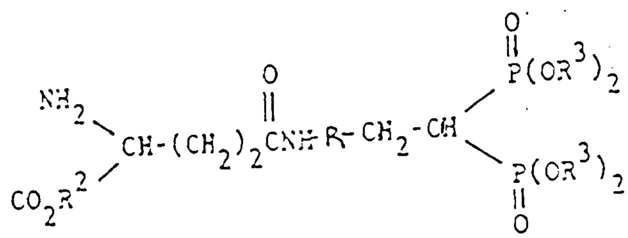

zur Durchführung des Verfahrens gemäß Anspruch 4 oder 6,
**dadurch gekennzeichnet,**
daß es aus folgendem besteht:

- Schutz der Aminfunktion des $\alpha$-Benzylesters der Glutaminsäure in Form eines Urethans;
- Peptidkopplung der besagten Aminosäure mit 2-Amino-4,4-bis(dialkylphosphon)alkylbutyrat-Derivaten in Gegenwart eines Kopplungsmittels, wie z.B. Dicyclohexylcarbodiimid;
- Entfernung des Schutzes der Aminfunktion durch Säurehydrolyse mit Trifluoressigsäure.

**8.** Verfahren zum Erhalten eines Synthons der Formel

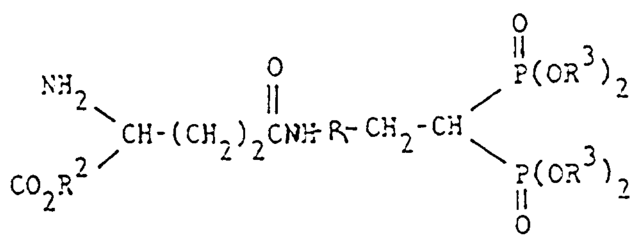

zur Durchführung des Verfahrens gemäß Anspruch 4 oder 6,
**dadurch gekennzeichnet,**
daß es in der Durchführung einer nukleophilen Michael-Addition eines Primäramids der Formel

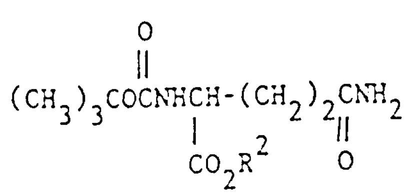

an das Tetraalkylvinylidendiphosphonat der Formel

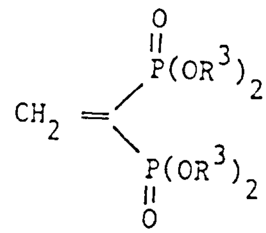

besteht.

**Patentansprüche für folgenden Vertragsstaat : GR**

1. Verfahren zur Herstellung von gem-Diphosphonsäure-Amethopterin- (Methotrexat-) und Deaza-N-10-amethopterin-Verbindungen in Form eines Amethopterin-Propharmakons, dessen geminale Diphosphonsäure-Gruppierung über eine Peptidbindung mit der Pteroylglutaminsäure-Gruppierung verbunden ist, und welches folgende Formel aufweist:

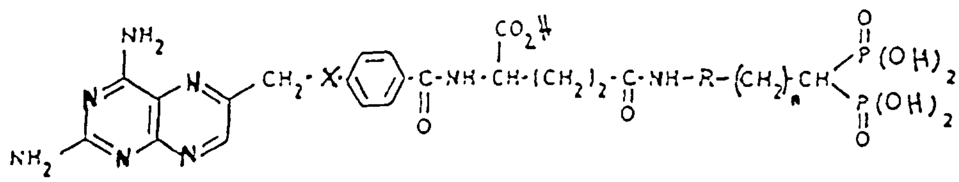

**gekennzeichnet durch** die Schritte, daß man
auf ein Zwischenprodukt

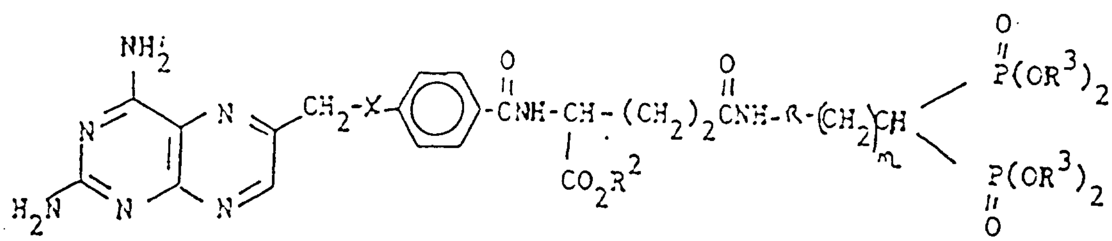

zuerst Trimethylsilylbromid einwirken läßt, gefolgt von einer Methanolyse zur Hydrolyse der Phosphonsäureester,
zweitens Soda einwirken läßt, um die Carboxylester zu hydrolysieren,
wobei in den Formeln
$n \geqq 0$, bevorzugt $n = 0, 1, 2, 3$ bedeutet
und wobei R entweder nicht vorhanden ist oder aus der Gruppe

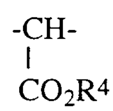

besteht,
X die Bedeutung

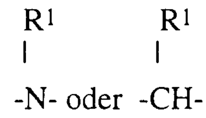

hat, und wobei
$R^1$, $R^3$, $R^4$, $R^5$ die Bedeutung H oder niedriges Alkyl ($C_1$ bis $C_6$) hat und
$R^2$ die Bedeutung H oder niedriges Alkyl ($C_1$ bis $C_6$) und Benzyl hat, wenn R vorhanden ist.

**2.** Verfahren zur Herstellung von gem-Diphosphonsäure-Amethopterin- (Metho-trexat-) und Deaza-N-10-amethopterin-Verbindungen in Form eines Amethopterin-Propharmakons, dessen geminale Diphosphonsäure-Gruppierung über eine Peptidbindung mit der Pteroylglutaminsäure-Gruppierung verbunden ist, und welches folgende Formel aufweist:

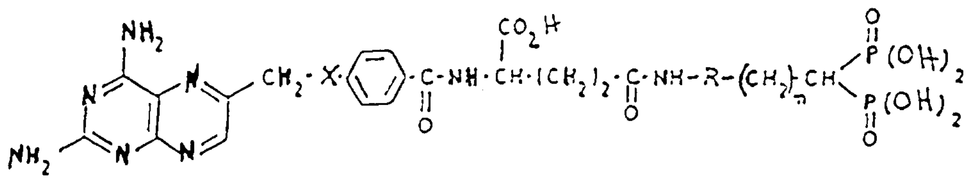

**gekennzeichnet durch** die Schritte, daß man
auf ein Zwischenprodukt der Formel

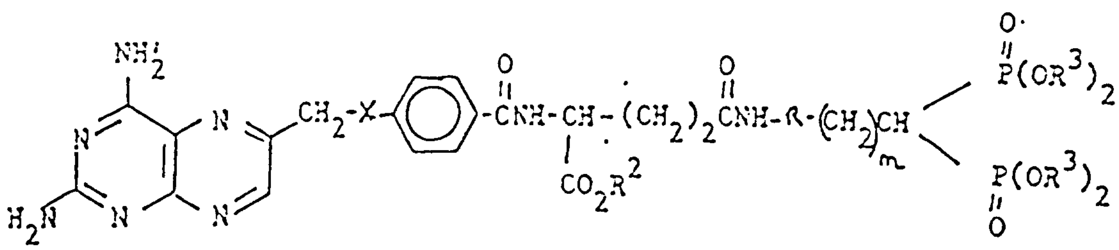

zuerst Bromwasserstoffsäure in Gegenwart von Essigsäure einwirken läßt, um die Phosphonsäureester zu hydrolysieren;
zweitens Soda einwirken laßt, um die Carboxylester zu hydrolysieren, wobei in den Formeln
$n \geqq 0$, bevorzugt $n = 0, 1, 2, 3$ bedeutet
und wobei R entweder nicht vorhanden ist oder aus der Gruppe

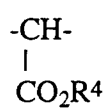

besteht,
X die Bedeutung

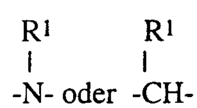

hat, und wobei
$R^1$, $R^3$, $R^4$, $R^5$ die Bedeutung H oder niedriges Alkyl ($C_1$ bis $C_6$) hat und

$R^2$ die Bedeutung H oder niedriges Alkyl ($C_1$ bis $C_6$) und Benzyl hat, wenn R vorhanden ist.

**3.** Verfahren nach Anspruch 1 oder 2, wobei
X die Bedeutung

-N-
|
$CH_3$

oder $-CH_2-$ hat.

**4.** Gem-Diphosphonsäure-Verbindungen, die insbesondere als Zwischenprodukte anzuwenden sind und die Formel

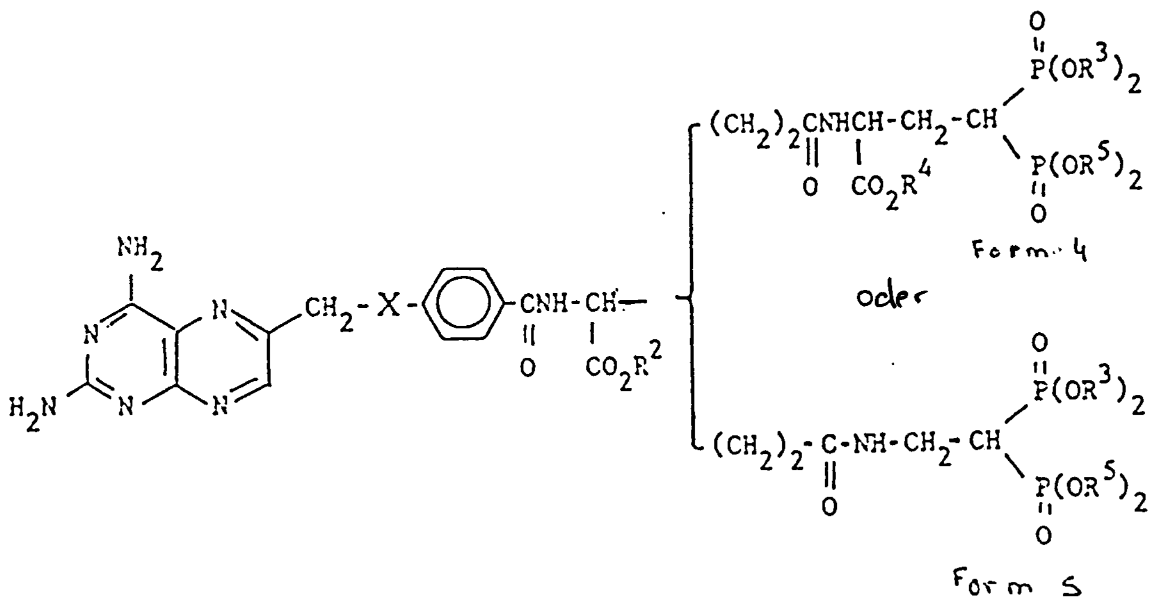
Form 4
oder
Form 5

aufweisen, wobei
X die Bedeutung

$R^1$ $R^1$
| |
-N- oder -CH-

hat, und wobei
$R^1$ die Bedeutung H oder niedriges Alkyl ($C_1$ bis $C_6$) hat,
$R^2$ die Bedeutung niedriges Alkyl ($C_1$ bis $C_6$) für die Form 5; Benzyl für die Form 4 hat,
$R^3$, $R^4$, $R^5$ die Bedeutung niedriges Alkyl ($C_1$ bis $C_6$) hat,
sowie deren Säure- und Salzderivate.

**5.** Gem-Diphosphonsäure-Verbindungen, die insbesondere als Zwischenprodukte anzuwenden sind und die Formel

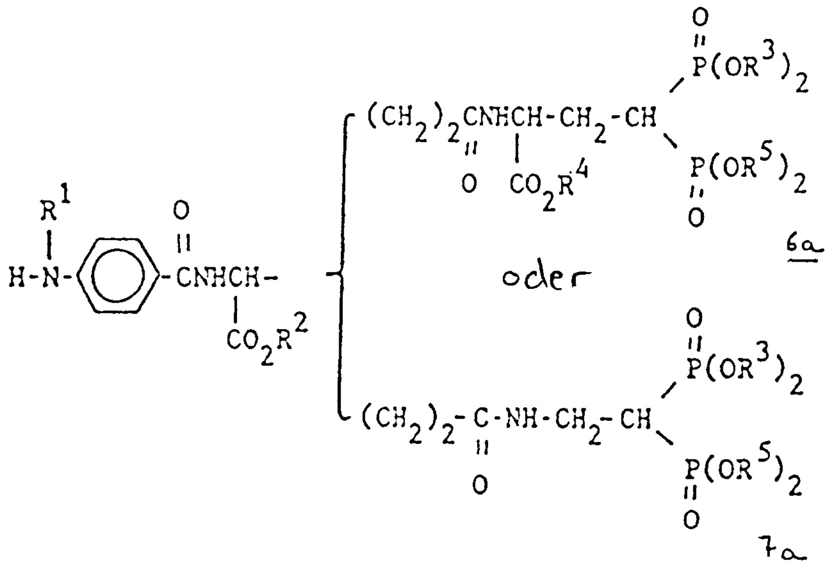

6a

oder

7a

aufweisen, wobei
$R^1$ die Bedeutung H oder niedriges Alkyl ($C_1$ bis $C_6$) hat,
$R^3$, $R^4$, $R^5$ die Bedeutung niedriges Alkyl ($C_1$ bis $C_6$) hat,
$R^2$ die Bedeutung niedriges Alkyl ($C_1$ bis $C_6$) für 7a, Benzyl für 6a hat,
sowie deren Säure- und Salzderivate.

**6.** Gem-Diphosphonsäure-Verbindungen, die insbesondere als Zwischenprodukte anzuwenden sind und die Formel

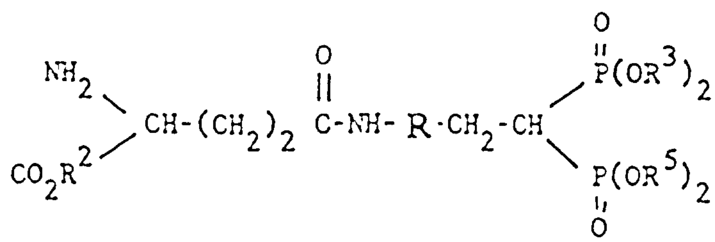

aufweisen, wobei
R nicht vorhanden ist oder

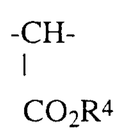

bedeutet,
$R^2$ die Bedeutung H oder niedriges Alkyl ($C_1$ bis $C_6$); Benzyl hat, wenn R vorhanden ist,
$R^3$, $R^4$, $R^5$ die Bedeutung niedriges Alkyl ($C_1$ bis $C_6$) hat,
sowie deren Säure- und Salzderivate.